# EUROPEAN PATENT SPECIFICATION

(11) **EP 3 263 601 B1**
(45) Date of publication and mention of the grant of the patent: **24.11.2021**
(21) Application number: 17182243.0
(22) Date of filing: 02.10.2013
(51) Int. Cl.: C07K 16/28, A61K 39/395, A61P 35/00

(54) **COMBINATION OF ANTI-KIR ANTIBODIES AND ANTI-PD-1 ANTIBODIES TO TREAT CANCER**
KOMBINATION AUS ANTI-KIR-ANTIKÖRPERN UND PD-1-ANTIKÖRPERN ZUR BEHANDLUNG VON KREBS
COMBINAISON D'ANTICORPS ANTI-KIR ET ANTI-PD-1 POUR TRAITER LE CANCER

(30) Priority: 02.10.2012 US 201261708784 P
(43) Date of publication of application: 03.01.2018
(62) Divisional of application: 13774596.4
(73) Proprietor: Bristol-Myers Squibb Company, Princeton, NJ 08543 (US)
(72) Inventor: GRAZIANO, Robert F., Princeton, NJ 08543 (US); GUPTA, Ashok, K., Princeton, NJ 08543 (US); KIM, Su Young, Princeton, NJ 08543 (US); WIGGINTON, Jon, Rockville, MD 20850 (US)
(74) Representative: Mewburn Ellis LLP

(56) References cited:
- WO-A1-2006/121168
- WO-A2-2006/003179
- WO-A2-2006/072625
- WO-A2-2012/160448
- Anonymous: "A Phase I Study of an Anti-KIR Antibody in Combination With an Anti-PD1 Antibody in Patients With Advanced Solid Tumors - Full Text View - ClinicalTrials.gov", , 24 October 2012 (2012-10-24), XP055089900, Retrieved from the Internet: URL:http://clinicaltrials.gov/show/NCT0171 4739 [retrieved on 2013-11-25]

## Description

### BACKGROUND

Natural killer (NK) cells constitute 15% of peripheral blood lymphocytes and play an important role in the ability of the innate immune system to fight off viral infections and also cancer (Purdy AK et al., Cancer Biol Ther 2009; 8:13-22). NK cells bind to target cells through multiple receptors, including natural cytotoxicity receptors (NCR), the Fc receptor CD 16, NKG2D, and others. Binding of ligand to receptor initiates tyrosine phosphorylation and recruitment of accessory signaling molecules. This cascade results in activation of the NK cell, release of preformed granules containing perforin and granzymes into the target cell, and apoptosis. The concurrent release of cytokines and chemokines results in a micro-environmental milieu that recruits other immune cells. NK cells have the capability of binding every cell in the body (Murphy WJ, et al., Biol Blood Marrow Transplant 2012; 18:S2-S7). However, binding of normal cells does not result in cytotoxic activity because of the ability of NK cells to simultaneously utilize a different set of receptors to bind major histocompatibility complex (MHC) class I molecules. Binding of human leukocyte antigen (HLA) E to the NKG2A/CD94 heterodimeric receptor, or of HLA-A, B and C molecules to inhibitory killer Ig-like receptors (KIRs), results in tyrosine phosphorylation, recruitment of the signaling adaptors SHP-1 or SHP-2, and downstream signaling. The end result is a dominant signal that suppresses normal activation signals. Thus, KIR/HLA interaction can impact NK cell responsiveness and also the development of the total number of mature responsive NK cells, known as licensing.

There are seven inhibitory KIRs and seven activating KIRs, which is one factor that results in diversity of KIR inheritance and expression. KIR is also expressed on natural killer T (NKT) cells and a small subset of T cells (Uhrberg M, et al., J. Immunol. 2001; 166:3923-3932). Thus, mechanistically, blockade of inhibitory KIR could induce anti-tumor effects by allowing for activation of NK cell and possibly also some T cells.

Evidence in support of NK cell involvement in the anti-tumor response comes from the hematopoietic stem cell transplant (HSCT) setting. Given the diversity in both KIR and HLA, it is not surprising that KIR on donor NK cells may not interact with host HLA, referred to as KIR mismatch. The finding that AML patients transplanted with KIR mismatched donor NK cells had lower relapse rates (3% versus 47%, p <0.01) and reduced risk of relapse (relative risk 0.48, 95% CI 0.29-0.78) gave scientific support for the role of NK cells in the anti-tumor response (Ruggeri L, et al. Blood. 2007;110:433-440).

In melanoma, certain KIR and HLA combinations may provide a more immunosuppressive environment, since certain combinations are seen more frequently in metastatic patients compared to non-metastatic patients (Naumova E, et al. Cancer Immunol Immunother 2005; 54:172-178). KIR mismatch has been shown to be a favorable prognostic marker for high risk neuroblastoma patients undergoing autologous HSCT (Delgado DC, et al., Cancer Res 2010; 70:9554-9561). Experimental support for the important role of NK cells in solid tumors comes from murine studies in which mice lacking T cells could still eradicate large solid tumors following NK cell activation by the addition of IL-15 (Liu RB, et al., Cancer Res 2012; 72:1964-1974).

Programmed Cell Death 1 (PD-1) is a cell surface signaling receptor that plays a critical role in the regulation of T cell activation and tolerance (Keir ME, et al., Annu Rev Immunol 2008; 26:677-704). It is a type I transmembrane protein and together with BTLA, CTLA-4, ICOS and CD28, comprise the CD28 family of T cell co-stimulatory receptors. PD-1 is primarily expressed on activated T cells, B cells, and myeloid cells (Dong H, et al., Nat Med 1999; 5:1365-1369). It is also expressed on natural killer (NK) cells (Terme M, et al., Cancer Res 2011; 71:5393-5399). Binding of PD-1 by its ligands, PD-L1 and PD-L2, results in phosphorylation of the tyrosine residue in the proximal intracellular immune receptor tyrosine inhibitory domain, followed by recruitment of the phosphatase SHP-2, eventually resulting in down-regulation of T cell activation. One important role of PD-1 is to limit the activity of T cells in peripheral tissues at the time of an inflammatory response to infection, thus limiting the development of autoimmunity (Pardoll DM., Nat Rev Cancer 2012; 12:252-264). Evidence of this negative regulatory role comes from the finding that PD-1 deficient mice develop lupus-like autoimmune diseases including arthritis and nephritis, along with cardiomyopathy (Nishimura H, et al., Immunity 1999; 11:141-151; and Nishimura H, et al., Science 2001; 291:319-322). In the tumor setting, the consequence is the development of immune resistance within the tumor microenvironment. PD-1 is highly expressed on tumor infiltrating lymphocytes, and its ligands are up-regulated on the cell surface of many different tumors (Dong H, et al., Nat Med 2002; 8:793-800). Multiple murine cancer models have demonstrated that binding of ligand to PD-1 results in immune evasion. In addition, blockade of this interaction results in anti-tumor activity (Topalian SL, et al., New Eng J Med 2012; 366(26):2443-2454; Topalian SL, et al., Curr Opin Immunol 2012; 24:207-212; Brahmer JR, et al., New Eng J Med 2012; 366(26):2455-2465; Hamid O, et al., New Eng J Med 2013; 369:134-144; Hamid O and Carvajal RD, Expert Opin Biol Ther 2013; 13(6):847-861). WO 2006/072625 discloses the combination therapy of anti-KIR antibodies with interleukin-2.

Patients with metastatic or refractory solid tumors have very poor prognosis (Rosenberg SA, et al., Cancer immunotherapy in Cancer: Principles & Practice of Oncology (Eds DeVita VT, Lawrence TS and Rosenberg SA) 2011; 332-344 (Lippincott Williams & Wilkins, Philadelphia PA)). Despite advances in multimodal therapy, increases in overall survival in this patient population have been limited. Accordingly, it is an object of the present invention to provide improved methods for treating subjects with such tumors (e.g., advanced refractory solid tumors).

### SUMMARY

Provided herein are methods for treating cancer, e.g., advanced refractory solid tumors, in a human patient, comprising administering to the patient a combination of an anti-KIR antibody and an anti-PD-1 antibody, wherein the combination is administered (or is for administration) according to a particular clinical dosage regimen (i.e., at a particular dose amount and according to a specific dosing schedule). In one non-limiting embodiment, the human patient suffers from a tumor (e.g., an advanced refractory solid tumor) selected from the group consisting of non-small cell lung cancer (NSCLC), renal cell carcinoma (RCC), melanoma, colorectal cancer, and serous ovarian carcinoma. Other tumors which can be treated are described in the following Detailed Description.

According to one aspect, the present invention relates to an anti-KIR antibody and an PD-1 antibody for use in treating cancer, wherein the anti-KIR antibody binds to KIR2DL1, KIR2DL2 and/or KIR2DL3 receptors and potentiates NK cell activity mediated by KIR2DL1, KIR2DL2 and/or KIR2DL3 receptors, and wherein the anti-PD-1 antibody blocks the inhibitory activity of PD-1.

According to a further aspect, the present invention relates to an anti-KIR antibody for use in treating cancer, wherein the treatment further comprises administration of an anti-PD-1 antibody, wherein the anti-KIR antibody binds to KIR2DL1, KIR2DL2 and/or KIR2DL3 receptors and potentiates NK cell activity mediated by KIR2DL1, KIR2DL2 and/or KIR2DL3 receptors, and wherein the anti-PD-1 antibody blocks the inhibitory activity of PD-1.

According to a further aspect, the present invention relates to an anti-PD-1 antibody for use in treating cancer, wherein the treatment further comprises administration of an anti-KIR antibody, wherein the anti-KIR antibody binds to KIR2DL1, KIR2DL2 and/or KIR2DL3 receptors and potentiates NK cell activity mediated by KIR2DL1, KIR2DL2 and/or KIR2DL3 receptors, and wherein the anti-PD-1 antibody blocks the inhibitory activity of PD-1.

An exemplary anti-KIR antibody is lirilumab (also previously referred to as BMS-986015 or IPH2102) comprising the heavy and light chains having the sequences shown in SEQ ID NOs:1 and 2, respectively, or antigen binding fragments and variants thereof. In other embodiments, the antibody comprises the heavy and light chain complementarity determining regions (CDRs) or variable regions (VRs) of lirilumab. Accordingly, in one embodiment, the antibody comprises the CDR1, CDR2, and CDR3 domains of the heavy chain variable (VH) region of lirilumab having the sequence shown in SEQ ID NO:3, and the CDR1, CDR2 and CDR3 domains of the light chain variable (VL) region of LIRILUMAB having the sequence shown in SEQ ID NO:5. In another embodiment, the antibody comprises CDR1, CDR2 and CDR3 heavy chain sequences as set forth in SEQ ID NOs:7, 8, and 9, respectively, and CDR1, CDR2 and CDR3 light chain sequences as set forth in SEQ ID NOs: 10, 11, and 12, respectively. In another embodiment, the antibody comprises VH and/or VL regions having the amino acid sequences set forth in SEQ ID NO:3 and/or SEQ ID NO:5, respectively. In another embodiment, the antibody comprises the VH and/or VL regions encoded by the nucleic acid sequences set forth in SEQ ID NO:4 and/or SEQ ID NO:6, respectively. In another embodiment, the antibody competes for binding with, and/or binds to the same epitope on KIR as, the above-mentioned antibodies. In another embodiment, the antibody has at least about 90% variable region amino acid sequence identity with the above-mentioned antibodies (e.g., at least about 90%, 95% or 99% variable region identity with SEQ ID NO:3 or SEQ ID NO:5).

An exemplary anti-PD-1 antibody is nivolumab (referred to as 5C4 in WO 2006/121168; also known as BMS-936558, MDX-1106 or ONO-4538) comprising heavy and light chains having the sequences shown in SEQ ID NOs:17 and 18, respectively, or antigen binding fragments and variants thereof. In other embodiments, the antibody comprises the heavy and light chain CDRs or VRs of nivolumab. Accordingly, in one embodiment, the antibody comprises the CDR1, CDR2, and CDR3 domains of the VH region of lirilumab having the sequence shown in SEQ ID NO:19, and the CDR1, CDR2 and CDR3 domains of the VL region of lirilumab having the sequence shown in SEQ ID NO:21. In another embodiment, the antibody comprises the heavy chain CDR1, CDR2 and CDR3 domains having the sequences set forth in SEQ ID NOs:23, 24, and 25, respectively, and the light chain CDR1, CDR2 and CDR3 domains having the sequences set forth in SEQ ID NOs: 26, 27, and 28, respectively. In another embodiment, the antibody comprises VH and/or VL regions having the amino acid sequences set forth in SEQ ID NO: 19 and/or SEQ ID NO:21, respectively. In another embodiment, the antibody comprises the heavy chain variable (VH) and/or light chain variable (VL) regions encoded by the nucleic acid sequences set forth in SEQ ID NO:20 and/or SEQ ID NO:22, respectively. In another embodiment, the antibody competes for binding with, and/or binds to the same epitope on PD-1 as, the above-mentioned antibodies. In another embodiment, the antibody has at least about 90% variable region amino acid sequence identity with the above-mentioned antibodies (e.g., at least about 90% , 95% or 99% variable region identity with SEQ ID NO:19 or SEQ ID NO:21).

Accordingly, in one aspect, methods of treating cancer (*e.g*., advanced refractory solid tumors) in a human patient are provided, the methods comprising administering to the patient, an effective amount of each of:
(a) an anti-KIR antibody comprising the CDR1, CDR2 and CDR3 domains in a heavy chain variable region having the sequence set forth in SEQ ID NO:3, and the CDR1, CDR2 and CDR3 domains in a light chain variable region having the sequence set forth in SEQ ID NO:5,
(b) an anti-PD-1 antibody comprising the CDR1, CDR2 and CDR3 domains in a heavy chain variable region having the sequence set forth in SEQ ID NO:19, and the CDR1, CDR2 and CDR3 domains in a light chain variable region having the sequence set forth in SEQ ID NO:21,
wherein the method comprises at least one administration cycle, wherein the cycle is a period of eight weeks, wherein for each of the at least one cycles, two doses of the anti-KIR antibody are administered at a dose of 0.1-20 mg/kg body weight and four doses of the anti-PD-1 antibody are administered at a dose of 0.1-20 mg/kg body weight.

In certain embodiments, each dose of the anti-KIR antibody is administered at 0.1, 0.3, 1, 3, 6, 10 or 20 mg/kg. In preferred embodiments, each dose of the anti-KIR antibody is administered at 0.3, 1 or 3 mg/kg.

In other embodiments, each dose of the anti-PD-1 antibody is administered at 0.1, 0.3, 1, 3, 6, 10 or 20 mg/kg body weight. In preferred embodiments, each dose of the anti-PD-1 antibody is administered at 0.3, 1, 3 or 10 mg/kg. In more preferred embodiments, the anti-PD-1 antibody is administered at a dose of 3 mg/kg.

In one embodiment, the anti-KIR antibody and anti-PD-1 antibody are administered at the following doses:
(a) 0.1 mg/kg anti-KIR antibody and 3 mg/kg of anti-PD-1 antibody;
(b) 0.3 mg/kg anti-KIR antibody and 3 mg/kg of anti-PD-1 antibody;
(c) 1 mg/kg anti-KIR antibody and 3 mg/kg of anti-PD-1 antibody;
(d) 3 mg/kg anti-KIR antibody and 3 mg/kg of anti-PD-1 antibody;
(e) 6 mg/kg anti-KIR antibody and 3 mg/kg of anti-PD-1 antibody; or
(f) 10 mg/kg anti-KIR antibody and 3 mg/kg of anti-PD-1 antibody.

Accordingly, in one embodiment, the dose of the anti-KIR and/or anti-PD-1 antibody is calculated per mg/kg body weight. However, in another embodiment, the dose of the anti-KIR and/or anti-PD-1 antibody is a flat-fixed dose that is fixed irrespective of the weight of the patient. For example, the anti-KIR and/or anti-PD-1 antibody may be administered at a fixed dose of 5, 20, 75, 200, 400, 750 or 1500 mg, without regard to the patient's weight. In certain embodiments, the administered dose of the anti-PD-1 antibody may be fixed at 200 mg, while the anti-KIR antibody is administered at a fixed dose of 5, 20, 75, 200, 400 or 750 mg. In another embodiment, dosage regimens are adjusted to provide the optimum desired response (*e.g.,* an effective response).

In another embodiment, the anti-PD-1 antibody is administered on Days 1, 15, 29, and 43 of each cycle. In another embodiment, the anti-KIR antibody is administered on Days 1 and 29 of each cycle. In another embodiment, the anti-PD-1 antibody is administered prior to administration of the anti-KIR antibody on Days 1 and 29. In another embodiment, the anti-KIR antibody is administered within 30 minutes of the anti-PD-1 antibody. In another embodiment, the treatment consists of up to 12 cycles.

In one embodiment, the anti-PD-1 antibody and anti-KIR antibody are administered as a first ("front") line of treatment (*e.g*., the initial or first treatment). In another embodiment, the anti-PD-1 antibody and anti-KIR antibody are administered as a second line of treatment (*e.g*., after initial treatment with the same or a different therapeutic, including after relapse and/or where the first treatment has failed).

The anti-KIR and anti-PD-1 antibodies can be administered to a subject by any suitable means. In one embodiment, the antibodies are formulated for intravenous administration. In another embodiment, the antibodies are administered simultaneously (e.g., in a single formulation or concurrently as separate formulations). Alternatively, in another embodiment, the antibodies are administered sequentially (e.g., as separate formulations).

The efficacy of the treatment methods provided herein can be assessed using any suitable means. In one embodiment, the treatment produces at least one therapeutic effect selected from the group consisting of reduction in size of a tumor, reduction in number of metastasic lesions over time, complete response, partial response, and stable disease.

Also provided are kits that include a pharmaceutical composition containing an anti-KIR antibody, such as lirilumab, and an anti-PD-1 antibody, such as nivolumab, and a pharmaceutically-acceptable carrier, in a therapeutically effective amount adapted for use in the methods described herein. In one embodiment, the kit comprises:
(a) a dose of an anti-KIR antibody comprising the CDR1, CDR2 and CDR3 domains in a heavy chain variable region having the sequence set forth in SEQ ID NO:3, and the CDR1, CDR2 and CDR3 domains in a light chain variable region having the sequence set forth in SEQ ID NO:5;
(b) a dose of an anti-PD-1 antibody comprising the CDR1, CDR2 and CDR3 domains in a heavy chain variable region having the sequence set forth in SEQ ID NO:19, and the CDR1, CDR2 and CDR3 domains in a light chain variable region having the sequence set forth in SEQ ID NO:21; and
(c) instructions for using the anti-KIR antibody and anti-PD-1 antibody in a method of the in the invention.

In another aspect, an anti-KIR antibody is provided, the anti-KIR antibody comprising the CDR1, CDR2 and CDR3 domains in a heavy chain variable region having the sequence set forth in SEQ ID NO:3, and the CDR1, CDR2 and CDR3 domains in a light chain variable region having the sequence set forth in SEQ ID NO:5, for co-administration with an anti-PD-1 antibody comprising the CDR1, CDR2 and CDR3 domains in a heavy chain variable region having the sequence set forth in SEQ ID NO:19, and the CDR1, CDR2 and CDR3 domains in a light chain variable region having the sequence set forth in SEQ ID NO:21, in at least one cycle, wherein for each cycle two doses of the anti-KIR antibody are administered at a dose of 0.1, 0.3, 1, 3, 6, or 10 mg/kg and four doses of the anti-PD-1 antibody are administered at a dose of 3 mg/kg.

In another aspect of the invention, the anti-PD-1 antibody in any of the aforementioned embodiments is replaced by, or combined with, an anti-PD-L1 or anti-PD-L2 antibody. Exemplary anti-PD-L1 antibodies are described in WO 2007/005874, WO 2010/077634 and WO 2011/066389, and exemplary anti-PD-L2 antibodies are described in WO 2004/007679. Accordingly, the invention also features methods, compositions and kits for treating tumors in human patients using the above-described clinically effective dosages of an anti-KIR antibody combined with the above-described clinically effective dosages of an anti-PD-1 antibody, wherein the dosage of the PD-1 antibody is replaced with the same dosage of an anti-PD-L1 or anti-PD-L2 antibody.

### BRIEF DESCRIPTION OF THE DRAWINGS

Figure 1 shows inhibition of tumor growth *in vivo* using a combination treatment of an anti-KIR antibody and an anti-PD-1 antibody in a murine solid tumor model.
Figure 2 is a schematic illustrating the parts of a phase I clinical trial.

### DETAILED DESCRIPTION

### I. Definitions

As used herein, the term "subject" or "patient" is a human cancer patient (e.g., a patient having a tumor, such as an advanced refractory solid tumor, or a hematological malignancy).

As used herein, "effective treatment" refers to treatment producing a beneficial effect, e.g., amelioration of at least one symptom of a disease or disorder. A beneficial effect can take the form of an improvement over baseline, i.e., an improvement over a measurement or observation made prior to initiation of therapy according to the method. A beneficial effect can also take the form of arresting, slowing, retarding, or stabilizing of a deleterious progression of a marker of solid tumor. Effective treatment may refer to alleviation of at least one symptom of a solid tumor. Such effective treatment may, e.g., reduce patient pain, reduce the size and/or number of lesions, may reduce or prevent metastasis of a tumor, and/or may slow tumor growth.

The term "effective amount" refers to an amount of an agent that provides the desired biological, therapeutic, and/or prophylactic result. That result can be reduction, amelioration, palliation, lessening, delaying, and/or alleviation of one or more of the signs, symptoms, or causes of a disease, or any other desired alteration of a biological system. In reference to solid tumors, an effective amount comprises an amount sufficient to cause a tumor to shrink and/or to decrease the growth rate of the tumor (such as to suppress tumor growth) or to prevent or delay other unwanted cell proliferation. In some embodiments, an effective amount is an amount sufficient to delay tumor development. In some embodiments, an effective amount is an amount sufficient to prevent or delay tumor recurrence. An effective amount can be administered in one or more administrations. The effective amount of the drug or composition may: (i) reduce the number of cancer cells; (ii) reduce tumor size; (iii) inhibit, retard, slow to some extent and may stop cancer cell infiltration into peripheral organs; (iv) inhibit (i.e., slow to some extent and may stop tumor metastasis; (v) inhibit tumor growth; (vi) prevent or delay occurrence and/or recurrence of tumor; and/or (vii) relieve to some extent one or more of the symptoms associated with the cancer. In one example, an "effective amount" is the amount of anti-KIR antibody and the amount of anti-PD-1 antibody, in combination, clinically proven to effect a significant decrease in cancer or slowing of progression of cancer, such as an advanced solid tumor.

As used herein, the terms "fixed dose", "flat dose" and "flat-fixed dose" are used interchangeably and refer to a dose that is administered to a patient without regard for the weight or body surface area (BSA) of the patient. The fixed or flat dose is therefore not provided as a mg/kg dose, but rather as an absolute amount of the agent (*e.g.,* the anti-KIR antibody and/or anti-PD-1 antibody).

As used herein, a "body surface area (BSA)-based dose" refers to a dose (*e.g.,* of the anti-KIR antibody and/or anti-PD-1 antibody) that is adjusted to the body-surface area (BSA) of the individual patient. A BSA-based dose may be provided as mg/kg body weight. Various calculations have been published to arrive at the BSA without direct measurement, the most widely used of which is the Du Bois formula (see Du Bois D, Du Bois EF (Jun 1916) Archives of Internal Medicine 17 (6): 863-71; and Verbraecken, J. et al. (Apr 2006). Metabolism — Clinical and Experimental 55 (4): 515-24). Other exemplary BSA formulas include the Mosteller formula (Mosteller RD. N Engl J Med., 1987; 317:1098), the Haycock formula (Haycock GB, et al., J Pediatr 1978, 93:62-66), the Gehan and George formula (Gehan EA, George SL, Cancer Chemother Rep 1970, 54:225-235), the Boyd formula (Current, JD (1998), The Internet Journal of Anesthesiology 2 (2); and Boyd, Edith (1935), University of Minnesota. The Institute of Child Welfare, Monograph Series, No. x. London: Oxford University Press), the Fujimoto formula (Fujimoto S, et al., Nippon Eiseigaku Zasshi 1968;5:443-50), the Takahira formula (Fujimoto S, et al., Nippon Eiseigaku Zasshi 1968;5:443-50), and the Schlich formula (Schlich E, et al., Ernährungs Umschau 2010;57:178-183).

The term "antibody" describes polypeptides comprising at least one antibody derived antigen binding site (e.g., VH/VL region or Fv, or CDR). Antibodies include known forms of antibodies. For example, the antibody can be a human antibody, a humanized antibody, a bispecific antibody, or a chimeric antibody. The antibody also can be a Fab, Fab'2, ScFv, SMIP, Affibody®, nanobody, or a domain antibody. The antibody also can be of any of the following isotypes: IgG1, IgG2, IgG3, IgG4, IgM, IgA1, IgA2, IgAsec, IgD, and IgE. The antibody may be a naturally occurring antibody or may be an antibody that has been altered (e.g., by mutation, deletion, substitution, conjugation to a non-antibody moiety). For example, an antibody may include one or more variant amino acids (compared to a naturally occurring antibody) which changes a property (e.g., a functional property) of the antibody. For example, numerous such alterations are known in the art which affect, e.g., half-life, effector function, and/or immune responses to the antibody in a patient. The term antibody also includes artificial polypeptide constructs which comprise at least one antibody-derived antigen binding site.

As used herein, a "Killer Ig-like Receptor", "Killer Inhibitory Receptor", or "KIR", refers to a protein or polypeptide encoded by a gene that is a member of the KIR gene family or by a cDNA prepared from such a gene. A detailed review of the KIR gene family, including the nomenclature of KIR genes and KIR gene products, and Genbank accession numbers for exemplary KIRs, is "The KIR Gene Cluster" by M. Carrington and P. Norman, available at the NCBI web-site called "Bookshelf' (accessible via the World-Wide Web (WWW) address ncbi.nlm.nih.gov/books). The sequences of human KIR genes and cDNAs, as well as their protein products, are available in public databases, including GenBank. Non-limiting exemplary GenBank entries of human KIRs have the following accession numbers: KIR2DL1: Genbank accession number U24076, NM_014218, AAR16197, or L41267; KIR2DL2: Genbank accession number U24075 or L76669; KIR2DL3: Genbank accession number U24074 or L41268; KIR2DL4: Genbank accession number X97229; KIR2DS1: Genbank accession number X89892; KIR2DS2: Genbank accession number L76667; KIR2DS3: Genbank accession number NM_012312 or L76670 (splice variant); KIR3DL1: Genbank accession number L41269; and KIR2DS4: Genbank accession number AAR26325. A KIR may comprise from 1 to 3 extracellular domains, and may have a long (*i.e.,* more than 40 amino acids) or short (*i.e.,* less than 40 amino acids) cytoplasmic tail. As previously described herein, these features determine the nomenclature of a KIR. Exemplary KIR2DL1, KIR2DL2, KIR2DL3, and KIR2DS4 molecules comprise polypeptides having the following respective amino acid sequences:
**KIR2DL1 extracellular domain:** 
HEGVHRKPSLLAHPGXLVKSEETVILQCWSDVMFEHFLLHREGMFNDTLRLI
GEHHDGVSKANFSISRMTQDLAGTYRCYGSVTHSPYQVSAPSDPLDIVIIGLY
EKPSLSAQXGPTVLAGENVTLSCSSRSSYDMYHLSREGEAHERRLPAGPKVN
GTFQADFPLGPATHGGTYRCFGSFHDSPYEWSKSSDPLLVSVTGNPSNSWPSP
TEPSSKTGNPRHLH (SEQ ID NO:13),
 where "X" at position 16 is P or R, and where "X" at position 114 is P or L, representing allelic variants.
**KIR2DL2 extracellular domain:** 
HEGVHRKPSLLAHPGRLVKSEETVILQCWSDVRFEHFLLHREGKFKDTLHLIG
EHHDGVSKANFSIGPMMQDLAGTYRCYGSVTHSPYQLSAPSDPLDIVITGLYE
KPSLSAQPGPTVLAGESVTLSCSSRSSYDMYHLSREGEAHECRFSAGPKVNGT
FQADFPLGPATHGGTYRCFGSFRDSPYEWSNSSDPLLVSVIGNPSNSWPSPTEP
SSKTGNPRHLH (SEQ ID NO:14)
**KIR2DL3 extracellular domain:** 
HEGVHRKPSLLAHPGPLVKSEETVILQCWSDVRFQHFLLHREGKFKDTLHLIG
EHHDGVSKANFSIGPMMQDLAGTYRCYGSVTHSPYQLSAPSDPLDIVITGLYE
KPSLSAQPGPTVLAGESVTLSCSSRSSYDMYHLSREGEAHERRFSAGPKVNGT
FQADFPLGPATHGGTYRCFGSFRDSPYEWSNSSDPLLVSVTGNPSNSWPSPTE
PSSETGNPRHLH (SEQ ID NO:15)
**KIR2DS4 extracellular domain:**

QEGVHRKPSFLALPGHLVKSEETVILQCWSDVMFEHFLLHREGKFNNTLHLIG
EHHDGVSKANFSIGPMMPVLAGTYRCYGSVPHSPYQLSAPSDPLDMV (SEQ
ID NO:16)

The term "KIR2DL2/3" refers to either or both of the KIR2DL2 and KIR2DL3 receptors. These two receptors have a very high homology, are encoded by allelic forms of the same gene, and are considered by the art to be functionally similar.

As used herein, the terms "Programmed Death 1," "Programmed Cell Death 1," "Protein PD-1," "PD-1," PD1," "PDCD1," "hPD-1" and "hPD-I" are used interchangeably, and include variants, isoforms, species homologs of human PD-1, and analogs having at least one common epitope with PD-1. The complete PD-1 sequence can be found under GenBank Accession No. U64863 (SEQ ID NO:29).

The protein Programmed Death 1 (PD-1) is an inhibitory member of the CD28 family of receptors, that also includes CD28, CTLA-4, ICOS and BTLA. PD-1 is expressed on activated B cells, T cells, and myeloid cells (Agata *et al., supra;* Okazaki et al. (2002) Curr. Opin. Immunol. 14: 391779-82; Bennett et al. (2003) J Immunol 170:711-8). The initial members of the family, CD28 and ICOS, were discovered by functional effects on augmenting T cell proliferation following the addition of monoclonal antibodies (Hutloff et al. (1999) Nature 397:263-266; Hansen et al. (1980) Immunogenics 10:247-260). PD-1 was discovered through screening for differential expression in apototic cells (Ishida et al. (1992) EMBO J 11:3887-95). The other members of the family, CTLA-4 and BTLA, were discovered through screening for differential expression in cytotoxic T lymphocytes and TH1 cells, respectively. CD28, ICOS and CTLA-4 all have an unpaired cysteine residue allowing for homodimerization. In contrast, PD-1 is suggested to exist as a monomer, lacking the unpaired cysteine residue characteristic in other CD28 family members.

The PD-1 gene is a 55 kDa type I transmembrane protein that is part of the Ig gene superfamily (Agata et al. (1996) Int Immunol 8:765-72). PD-1 contains a membrane proximal immunoreceptor tyrosine inhibitory motif (ITIM) and a membrane distal tyrosine-based switch motif (ITSM) (Thomas, M.L. (1995) J Exp Med 181:1953-6; Vivier, E and Daeron, M (1997) Immunol Today 18:286-91). Although structurally similar to CTLA-4, PD-1 lacks the MYPPPY motif that is critical for B7-1 and B7-2 binding. Two ligands for PD-1 have been identified, PD-L1 and PD-L2, that have been shown to downregulate T cell activation upon binding to PD-1 (Freeman et al. (2000) J Exp Med 192:1027-34; Latchman et al. (2001) Nat Immunol 2:261-8; Carter et al. (2002) Eur J Immunol 32:634-43). Both PD-L1 and PD-L2 are B7 homologs that bind to PD-1, but do not bind to other CD28 family members. PD-L1 is abundant in a variety of human cancers (Dong et al. (2002) Nat. Med. 8:787-9). The interaction between PD-1 and PD-L1 results in a decrease in tumor infiltrating lymphocytes, a decrease in T-cell receptor mediated proliferation, and immune evasion by the cancerous cells (Dong et al. (2003) J. Mol. Med. 81:281-7; Blank et al. (2005) Cancer Immunol. Immunother. 54:307-314; Konishi et al. (2004) Clin. Cancer Res. 10:5094-100). Immune suppression can be reversed by inhibiting the local interaction of PD-1 with PD-L1, and the effect is additive when the interaction of PD-1 with PD-L2 is blocked as well (Iwai et al. (2002) Proc. Nat'l. Acad. Sci. USA 99:12293-7; Brown et al. (2003) J. Immunol. 170:1257-66).

Consistent with PD-1 being an inhibitory member of the CD28 family, PD-1 deficient animals develop various autoimmune phenotypes, including autoimmune cardiomyopathy and a lupus-like syndrome with arthritis and nephritis (Nishimura et al. (1999) Immunity 11:141-51; Nishimura et al. (2001) Science 291:319-22). Additionally, PD-1 has been found to play a role in autoimmune encephalomyelitis, systemic lupus erythematosus, graft-versus-host disease (GVHD), type I diabetes, and rheumatoid arthritis (Salama et al. (2003) J Exp Med 198:71-78; Prokunina and Alarcon-Riquelme (2004) Hum Mol Genet 13:R143; Nielsen et al. (2004) Lupus 13:510). In a murine B cell tumor line, the ITSM of PD-1 was shown to be essential to block BCR-mediated Ca²⁺-flux and tyrosine phosphorylation of downstream effector molecules (Okazaki et al. (2001) PNAS 98:13866-71).

### IIa. Anti-KIR Antibodies

Anti-human-KIR antibodies (or VH/VL domains derived therefrom) suitable for use in the invention can be generated using methods well known in the art. Alternatively, art recognized anti-KIR antibodies can be used. In preferred embodiments, the anti-KIR antibody is cross-reactive with multiple inhibitory KIR receptors and potentiates the cytotoxicity of NK cells bearing one or more of these receptors. For example, the anti-KIR antibody may bind to each of KIR2D2DL1, KIR2DL2, and KIR2DL3, and potentiate NK cell activity by reducing, neutralizing and/or reversing inhibition of NK cell cytotoxicity mediated by any or all of these KIRs. In further embodiments, the anti-KIR antibody does not bind KIR2DS4 and/or KIR2DS3. For example, monoclonal antibodies 1-7F9 (also known as IPH2101), 14F1, 1-6F1 and 1-6F5, described in WO 2006/003179 can be used. Antibodies that compete with any of these art-recognized antibodies for binding to KIR also can be used. Additional art-recognized anti-KIR antibodies which can be used include, for example, those disclosed in WO 2005/003168, WO 2005/009465, WO 2006/072625, WO 2006/072626, WO 2007/042573, WO 2008/084106, WO 2010/065939, WO 2012/071411 and WO/2012/160448.

An exemplary anti-KIR antibody is lirilumab (also referred to as BMS-986015, IPH2102, or in WO 2008/084106 as 1-7F9(S241P)) comprising heavy and light chains having the sequences shown in SEQ ID NOs:1 and 2, respectively, or antigen binding fragments and variants thereof. lirilumab is a fully human anti-KIR antibody that comprises the same heavy and light chain variable regions as 1-7F9 (described in WO 2006/003179), and thus binds to the same epitope as 1-7F9, but differs from 1-7F9 in that (1) it is prepared in Chinese hamster ovary (CHO) cells, whereas 1-7F9 is prepared from hybridoma cells, and (2) a stabilizing hinge mutation (S231P) has been introduced into lirilumab (WO 2008/084106).

In other embodiments, the antibody comprises the heavy and light chain CDRs or variable regions of lirilumab. Accordingly, in one embodiment, the antibody comprises the CDR1, CDR2, and CDR3 domains of the VH region of lirilumab having the sequence set forth in SEQ ID NO:3, and the CDR1, CDR2 and CDR3 domains of the VL region of lirilumab having the sequence set forth in SEQ ID NO:5. In another embodiment, the antibody comprises heavy chain CDR1, CDR2 and CDR3 domains having the sequences set forth in SEQ ID NOs:7, 8, and 9, respectively, and light chain CDR1, CDR2 and CDR3 domains having the sequences set forth in SEQ ID NOs:10, 11, and 12, respectively. In another embodiment, the antibody comprises VH and/or VL regions having the amino acid sequences set forth in SEQ ID NO:3 and/or SEQ ID NO: 5, respectively. In another embodiment, the antibody comprises the heavy chain variable (VH) and/or light chain variable (VL) regions encoded by the nucleic acid sequences set forth in SEQ ID NO:4 and/or SEQ ID NO:6, respectively. In another embodiment, the antibody competes for binding with, and/or binds to the same epitope on KIR as, the above-mentioned antibodies. In another embodiment, the antibody has at least about 90% variable region amino acid sequence identity with the above-mentioned antibodies (e.g., at least about 90%, 95% or 99% variable region identity with SEQ ID NO:3 or SEQ ID NO:5).

### IIb. Anti-PD-1 Antibodies

Anti-human-PD-1 antibodies (or VH and/or VL domains derived therefrom) suitable for use in the invention can be generated using methods well known in the art. Alternatively, art recognized anti-PD-1 antibodies can be used. For example, monoclonal antibodies 5C4 (referred to herein as nivolumab), 17D8, 2D3, 4H1, 4A11, 7D3, and 5F4, described in WO 2006/121168 can be used. Other known PD-1 antibodies include Lambrolizumab (MK-3475), described as h409A11 in WO 2008/156712, and AMP-514 described in WO 2012/145493. Further known PD-1 antibodies and other PD-1 inhibitors include those described in WO 2009/014708 and WO 2009/114335. Antibodies that compete with any of these art-recognized antibodies for binding to PD-1 also can be used.

An exemplary anti-PD-1 antibody is nivolumab comprising heavy and light chains having the sequences shown in SEQ ID NOs:17 and 18, respectively, or antigen binding fragments and variants thereof. In other embodiments, the antibody comprises the heavy and light chain CDRs or variable regions of nivolumab. Accordingly, in one embodiment, the antibody comprises the CDR1, CDR2, and CDR3 domains of the VH of nivolumab having the sequence set forth in SEQ ID NO:19, and the CDR1, CDR2 and CDR3 domains of the VL of nivolumab having the sequences set forth in SEQ ID NO:21. In another embodiment, the antibody comprises heavy chain CDR1, CDR2 and CDR3 domains having the sequences set forth in SEQ ID NOs:23, 24, and 25, respectively, and light chain CDR1, CDR2 and CDR3 domains having the sequences set forth in SEQ ID NOs:26, 27, and 28, respectively. In another embodiment, the antibody comprises VH and/or VL regions having the amino acid sequences set forth in SEQ ID NO: 19 and/or SEQ ID NO: 21, respectively. In another embodiment, the antibody comprises the heavy chain variable (VH) and/or light chain variable (VL) regions encoded by the nucleic acid sequences set forth in SEQ ID NO:20 and/or SEQ ID NO:22, respectively. In another embodiment, the antibody competes for binding with and/or binds to the same epitope on PD-1 as the above-mentioned antibodies. In another embodiment, the antibody has at least about 90% variable region amino acid sequence identity with the above-mentioned antibodies (e.g., at least about 90%, 95% or 99% variable region identity with SEQ ID NO:19 or SEQ ID NO:21).

### III. Pharmaceutical Compositions

Pharmaceutical compositions suitable for administration to human patients are typically formulated for parenteral administration, e.g., in a liquid carrier, or suitable for reconstitution into liquid solution or suspension for intravenous administration.

In general, such compositions typically comprise a pharmaceutically acceptable carrier. As used herein, the term "pharmaceutically acceptable" means approved by a government regulatory agency or listed in the U.S. Pharmacopeia or another generally recognized pharmacopeia for use in animals, particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil, glycerol polyethylene glycol ricinoleate, and the like. Water or aqueous solution saline and aqueous dextrose and glycerol solutions may be employed as carriers, particularly for injectable solutions (e.g., comprising an anti-KIR or anti-PD-1 antibody). Liquid compositions for parenteral administration can be formulated for administration by injection or continuous infusion. Routes of administration by injection or infusion include intravenous, intraperitoneal, intramuscular, intrathecal and subcutaneous. In one embodiment, the anti-KIR and/or anti-PD-1 antibodies are administered intravenously (e.g., separately or together, each, e.g., over the course of one hour, 90 minutes, or two hours).

### IV. Patient Populations

Provided herein are effective methods for treating cancer (e.g., advanced refractory solid tumors or hematological malignancies) in a human patient using a combination of an anti-KIR antibody and an anti-PD-1 antibody.

Because these methods operate by enhancing an immune response by blocking inhibitory receptors on T cells and NK cells, they are applicable to a very broad range of cancers. In one embodiment, the human patient suffers from non-small cell lung cancer (NSCLC), renal cell carcinoma (RCC), melanoma (e.g., cutaneous or intraocular malignant melanoma), colorectal cancer, or serous ovarian carcinoma. Examples of additional cancers that may be treated using a combination of an anti-PD-1 antibody and an anti-KIR antibody include liver cancer, bone cancer, pancreatic cancer, skin cancer, cancer of the head or neck, breast cancer, lung cancer, uterine cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B-lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combinations of said cancers. The present invention is also applicable to treatment of metastatic cancers.

Patients can be tested or selected for one or more of the above described clinical attributes prior to, during or after treatment.

### V. Combination Therapy

Combination therapies provided herein involve administration of an anti-KIR antibody and another antibody that blocks an inhibitory immune receptor (e.g., a receptor, which upon binding to its natural ligand, inhibits/neutralizes activity, such as cytotoxic activity), such as an anti-PD-1 antibody, to treat subjects afflicted with cancer (e.g., advanced refractory solid tumors).

In one embodiment, the invention provides an anti-KIR antibody and an anti-PD-1 antibody in combination to treat subjects having a solid tumor (e.g., an advanced refractory solid tumor). In a particular embodiment, the anti-KIR antibody is lirilumab. In another embodiment, the anti-PD-1 antibody is nivolumab.

As used herein, adjunctive or combined administration (coadministration) includes simultaneous administration of the compounds in the same or different dosage form, or separate administration of the compounds (e.g., sequential administration). Thus, the anti-KIR and anti-PD-1 antibodies can be simultaneously administered in a single formulation. Alternatively, the anti-KIR and anti-PD-1 antibodies can be formulated for separate administration and are administered concurrently or sequentially.

For example, the anti-PD1 antibody can be administered first followed by (e.g., immediately followed by) the administration of the anti-KIR antibody, or vice versa. In one embodiment, the anti-PD-1 antibody is administered prior to administration of the anti-KIR antibody on Days 1 and 29. In another embodiment, the anti-KIR antibody is administered within 30 minutes of the anti-PD-1 antibody. Such concurrent or sequential administration preferably results in both antibodies being simultaneously present in treated patients.

### VI. Treatment Protocols

Suitable treatment protocols for treating a human patient afflicted with cancer include, for example, administering to the patient an effective amount of each of:
(a) an anti-KIR antibody comprising the CDR1, CDR2 and CDR3 domains in a heavy chain variable region having the sequence set forth in SEQ ID NO:3, and the CDR1, CDR2 and CDR3 domains in a light chain variable region having the sequence set forth in SEQ ID NO:5,
(b) an anti-PD-1 antibody comprising the CDR1, CDR2 and CDR3 domains in a heavy chain variable region having the sequence set forth in SEQ ID NO:19, and the CDR1, CDR2 and CDR3 domains in a light chain variable region having the sequence set forth in SEQ ID NO:21,
wherein the method comprises at least one administration cycle, wherein the cycle is a period of eight weeks, wherein for each of the at least one cycles, two doses of the anti-KIR antibody are administered at a dose of 0.1-20 mg/kg body weight and four doses of the anti-PD-1 antibody are administered at a dose of 0.1-20 mg/kg body weight.

In certain embodiments, each dose of the anti-KIR antibody is administered at 0.1, 0.3, 1, 3, 6, 10 or 20 mg/kg. In preferred embodiments, each dose of the anti-KIR antibody is administered at 0.3, 1 or 3 mg/kg.

In other embodiments, each dose of the anti-PD-1 antibody is administered at 0.1, 0.3, 1, 3, 6, 10 or 20 mg/kg body weight. In preferred embodiments, each dose of the anti-PD-1 antibody is administered at 0.3, 1, 3 or 10 mg/kg. In more preferred embodiments, the anti-PD-1 antibody is administered at a dose of 3 mg/kg.

In one embodiment, the anti-KIR antibody and anti-PD-1 antibody are administered at the following doses:
(a) 0.1 mg/kg anti-KIR antibody and 3 mg/kg of anti-PD-1 antibody;
(b) 0.3 mg/kg anti-KIR antibody and 3 mg/kg of anti-PD-1 antibody;
(c) 1 mg/kg anti-KIR antibody and 3 mg/kg of anti-PD-1 antibody;
(d) 3 mg/kg anti-KIR antibody and 3 mg/kg of anti-PD-1 antibody;
(e) 6 mg/kg anti-KIR antibody and 3 mg/kg of anti-PD-1 antibody; or
(f) 10 mg/kg anti-KIR antibody and 3 mg/kg of anti-PD-1 antibody.

In another embodiment, the dose of the anti-KIR and/or anti-PD-1 antibody is varied over time. For example, the anti-KIR antibody and/or anti-PD-1 antibody may be initially administered at a high dose and may be lowered over time. In another embodiment, the anti-KIR antibody and/or anti-PD-1 antibody is initially administered at a low dose and increased over time.

In another embodiment, the amount of the anti-KIR and/or anti-PD-1 antibodies administered is constant for each dose. In another embodiment, the amount of antibody administered varies with each dose. For example, the maintenance (or follow-on) dose of the antibody can be higher or the same as the loading dose which is first administered. In another embodiment, the maintenance dose of the antibody can be lower or the same as the loading dose.

In another embodiment, the anti-KIR and/or anti-PD-1 antibodies are formulated for intravenous administration. In one embodiment, the anti-PD-1 antibody is administered on Days 1, 15, 29, and 43 of each cycle. In another embodiment, the anti-KIR antibody is administered on Days 1 and 29 of each cycle.

In other embodiments, the anti-KIR and/or anti-PD-1 antibodies are administered once per week, once every or three two weeks, once per month or as long as a clinical benefit is observed or until there is a complete response, confirmed progressive disease or unmanageable toxicity.

In another embodiment, a cycle of administration is eight weeks, which can be repeated, as necessary. In another embodiment, the treatment consists of up to 12 cycles.

In another embodiment, 4 doses of the anti-PD-1 antibody are administered per eight week cycle. In another embodiment, 2 doses of the anti-KIR antibody are administered per eight week cycle.

In another embodiment, the anti-PD-1 antibody and anti-KIR antibody are administered as a first line of treatment (*e.g*., the initial or first treatment). In another embodiment, the anti-PD-1 antibody and anti-KIR antibody are administered as a second line of treatment (*e.g*., after the initial or first treatment, including after relapse and/or where the first treatment has failed).

In another aspect, the invention features any of the aforementioned embodiments, wherein the anti-PD-1 antibody is replaced by, or combined with, an anti-PD-L1 or anti-PD-L2 antibody.

### VII. Outcomes

With respect to target lesions, responses to therapy may include:

| | |
|---|---|
| Complete Response (CR) (RECIST V1.1) | Disappearance of all target lesions. Any pathological lymph nodes (whether target or non-target) must have reduction in short axis to < 10 mm. |
| Partial Response (PR) (RECIST V1.1) | At least a 30% decrease in the sum of the diameters of target lesions, taking as reference the baseline sum diameters. |
| Progressive Disease (PD) (RECIST V1.1) | At least a 20% increase in the sum of the diameters of target lesions, taking as reference the smallest sum on study (this includes the baseline sum if that is the smallest on study). In addition to the relative increase of 20%, the sum must also demonstrate an absolute increase of at least 5 mm. (Note: the appearance of one or more new lesions is also considered progression). |
| Stable Disease (SD) (RECIST V1.1) | Neither sufficient shrinkage to qualify for PR nor sufficient increase to qualify for PD, taking as reference the smallest sum diameters while on study. |
| Immune-related Complete Response (irCR) (irRECIST) | Disappearance of all target lesions. Any pathological lymph nodes (whether target or non-target) must have reduction in short axis to < 10 mm. |
| Immune-related Partial Response (irPR) (irRECIST) | At least a 30% decrease in the sum of diameters of target lesions and all new measurable lesions (i.e., Percentage Change in Tumor Burden), taking as reference the baseline sum diameters. Note: the appearance of new measurable lesions is factored into the overall Tumor Burden, but does not automatically qualify as progressive disease until the sum of the diameters increases by ≥ 20% when compared to nadir. |
| Immune-related Progressive Disease (irPD) (irRECIST) | At least a 20% increase in Tumor Burden (i.e., the sum of diameters of target lesions, and any new measurable lesions) taking as reference the smallest sum on study (this includes the baseline sum if that is the smallest on study). In addition to the relative increase of 20%, the sum must also demonstrate an absolute increase of at least 5 mm. Tumor assessments using immune-related criteria for progressive disease incorporates the contribution of new measurable lesions. Each net percentage change in tumor burden per assessment accounts for the size and growth kinetics of both old and new lesions as they appear. |
| Immune-related Stable Disease (irSD) (irRECIST) | Neither sufficient shrinkage to qualify for irPR nor sufficient increase to qualify for irPD, taking as reference the smallest sum diameters while on study. |

With respect to non-target lesions, responses to therapy may include:

| | |
|---|---|
| Complete Response (CR) (RECIST V1.1) | Disappearance of all non-target lesions. All lymph nodes must be non-pathological in size (<10 mm short axis). |
| Non-CR/Non-PD (RECIST V1.1) | Persistence of one or more non-target lesion(s). |
| Progressive Disease (PD) (RECIST V1.1) | Unequivocal progression of existing non-target lesions. The appearance of one or |
| | more new lesions is also considered progression. |
| Immune-related Complete Response (irCR) (irRECIST) | Disappearance of all non-target lesions. All lymph nodes must be non-pathological in size (< 10 mm short axis). |
| Immune-related Progressive Disease (irPD) (irRECIST) | Increases in number or size of non-target lesion(s) does not constitute progressive disease unless/until Tumor Burden increases by 20% (ie the sum of the diameters at nadir of target lesions and any new measurable lesions increases by the required amount). Non-target lesions are not considered in the definition of Stable Disease and Partial Response. |

Patients treated according to the methods disclosed herein preferably experience improvement in at least one sign of cancer. In one embodiment, improvement is measured by a reduction in the quantity and/or size of measurable tumor lesions. In another embodiment, lesions can be measured on chest x-rays or CT or MRI films. In another embodiment, cytology or histology can be used to evaluate responsiveness to a therapy.

In one embodiment, the patient treated exhibits a complete response (CR), a partial response (PR), stable disease (SD), immune-related complete disease (irCR), immune-related partial response (irPR), or immune-related stable disease (irSD). In another embodiment, the patient treated experiences tumor shrinkage and/or decrease in growth rate, i.e., suppression of tumor growth. In another embodiment, unwanted cell proliferation is reduced or inhibited. In yet another embodiment, one or more of the following can occur: the number of cancer cells can be reduced; tumor size can be reduced; cancer cell infiltration into peripheral organs can be inhibited, retarded, slowed, or stopped; tumor metastasis can be slowed or inhibited; tumor growth can be inhibited; recurrence of tumor can be prevented or delayed; one or more of the symptoms associated with cancer can be relieved to some extent.

In other embodiments, administration of effective amounts of the anti-KIR antibody and anti-PD-1 antibody according to any of the methods provided herein produces at least one therapeutic effect selected from the group consisting of reduction in size of a tumor, reduction in number of metastatic lesions appearing over time, complete remission, partial remission, or stable disease. In still other embodiments, the methods of treatment produce a comparable clinical benefit rate (CBR = CR+ PR+ SD ≥ 6 months) better than that achieved by an anti-KIR antibody or anti-PD-1 antibody alone. In other embodiments, the improvement of clinical benefit rate is about 20% 20%, 30%, 40%, 50%, 60%, 70%, 80% or more compared to an anti-KIR antibody or anti-PD-1 antibody alone.

### VIII. Kits and Unit Dosage Forms

Also provided herein are kits which include a pharmaceutical composition containing an anti-KIR antibody, such as lirilumab, and an anti-PD-1 antibody, such as nivolumab, and a pharmaceutically-acceptable carrier, in a therapeutically effective amount adapted for use in the preceding methods. The kits optionally also can include instructions, e.g., comprising administration schedules, to allow a practitioner (e.g., a physician, nurse, or patient) to administer the composition contained therein to administer the composition to a patient having cancer (e.g., a solid tumor). The kit also can include a syringe.

Optionally, the kits include multiple packages of the single-dose pharmaceutical compositions each containing an effective amount of the anti-KIR or anti-PD-1 antibody for a single administration in accordance with the methods provided above. Instruments or devices necessary for administering the pharmaceutical composition(s) also may be included in the kits. For instance, a kit may provide one or more pre-filled syringes containing an amount of the anti-KIR or anti-PD-1 antibody.

In one embodiment, the present invention provides a kit for treating a cancer in a human patient, the kit comprising:
(a) a dose of an anti-KIR antibody comprising the CDR1, CDR2 and CDR3 domains in a heavy chain variable region having the sequence set forth in SEQ ID NO:3, and the CDR1, CDR2 and CDR3 domains in a light chain variable region having the sequence set forth in SEQ ID NO:5;
(b) a dose of an anti-PD-1 antibody comprising the CDR1, CDR2 and CDR3 domains in a heavy chain variable region having the sequence set forth in SEQ ID NO:19, and the CDR1, CDR2 and CDR3 domains in a light chain variable region having the sequence set forth in SEQ ID NO:21; and
(c) instructions for using the anti-KIR antibody and anti-PD-1 antibody in the methods described herein.

The following examples are merely illustrative and should not be construed as limiting the scope of this disclosure in any way.

### EXAMPLES

### Example 1: Pre-Clinical Pharmacology of Anti-PD-1 Antibody (nivolumab)

Nivolumab is a fully human, IgG4 (kappa) isotype monoclonal antibody that binds to PD-1 with high affinity and specificity, thus precluding binding to its ligands PD-L1 and PD-L2 (see WO 2006/121168). The K_{D} for binding of nivolumab to PD-1 has been determined to be about 10⁻⁹ M as measured by surface plasmon resonance (Biacore) analysis (see WO 2006/121168), and about 2.9 × 10⁻¹² M as determined by bio-layer interferometry (ForteBio). Nivolumab does not bind other related family members, such as BTLA, CTLA-4, ICOS or CD28. Pre-clinical testing of nivolumab demonstrated that binding to PD-1 results in enhanced T cell proliferation and release of interferon-gamma (IFN-gamma) *in vitro* (see WO 2006/121168). The heavy and light chain amino acid sequences of nivolumab are provided in SEQ ID NOs:17 and 18, respectively.

### Example 2: Low Toxicity of Anti-PD-1 Antibody (nivolumab) In Vivo

Toxicology studies in cynomolgus monkeys confirmed that nivolumab was well tolerated at doses up to 50 mg/kg given twice weekly for 27 doses. Drug-related findings were limited to a reversible decrease in triiodothyronine (T3) by 28%, without concomitant abnormalities in other markers of thyroid function (data not shown).

### Example 3: Clinical Pharmacology and Safety of Anti-PD-1 Antibodies

As of May 2011, 273 subjects had been treated with nivolumab in four Phase I studies. One was a study of subjects with active hepatitis C infection, two were dose escalation studies in subjects with advanced malignancies, and the other was a combination study with Ipilimumab. A total of 273 subjects received one or more doses of nivolumab at doses from 0.3 to 10 mg/kg. No maximum tolerated dose (MTD) was reached. There was no pattern of incidence, severity or relationship of adverse events (AEs) with the dose or with the tumor type. Twenty-three subjects (8.4%) had serious adverse events (SAEs) related to nivolumab.

In one study (CA209001), 39 subjects received a single dose of nivolumab at 0.3, 1, 3, or 10 mg/kg with an opportunity for retreatment at three months. All subjects had at least one AE, and of those, 35 (88%) were related to treatment. The most frequent AEs regardless of causality were fatigue (56%), nausea (44%), proteinuria (38%), constipation (33%), back pain (33%), dry mouth (28%), vomiting (28%), rash (26%), dyspnea (26%), and anorexia (23%). Treatment related AEs were reported in 35 of 39 (90%) of subjects. Of those, 11 experienced grade 3 AEs, and one subject had a grade 4 decreased lymphocyte count. There were 68 SAEs and three were related to treatment (grade 2 anemia, grade 2 hypothyroidism, and grade 3 colitis). Among 12 deaths, none were considered related to nivolumab.

In a larger Phase I study (CA209003) that is still ongoing, 169 subjects received multiple doses of nivolumab at 0.1, 0.3, 1, 3 and 10 mg/kg, at an interval of every two weeks. One hundred forty (83%) subjects reported at least one AE, the most common of which did not differ markedly from those listed above. This was consistent with the safety experience observed in the single dose administration of nivolumab. The most common treatment related AEs were fatigue (22%), rash (15%), pruritus (11%), diarrhea (9%) and nausea (8%). Sixty-five (38%) subjects experienced grade 3 or 4 AEs, and of those, 23 subjects had AEs related to treatment. Fifty-eight (34%) subjects reported SAEs, all of which occurred in the 1, 3 or 10 mg/kg treatment groups, and of those 16 (9%) subjects had SAEs that were related to treatment. Types of treatment related SAEs included endocrinopathies (hyperthyroidism, hypophysitis, secondary adrenocortical insufficiency, increased lipase), gastrointestinal toxicities (abdominal pain, nausea, vomiting, dehydration, diarrhea, colitis), hepatotoxicities (hepatitis, increased ALT, AST and alkaline phosphatase), pulmonary toxicities (dyspnea, pneumonitis, acute respiratory distress syndrome), and other toxicities (fatigue, cellulitis, infusion related reaction, myoclonus, malignant neoplasm, myelodysplastic syndrome). As of November 30, 2011, 33 deaths have been reported; two subjects at 0.1 mg/kg dose, eight subjects at 1 mg/kg dose, three subjects at 3 mg/kg dose, and 20 subjects at 10 mg/kg dose. Thirty deaths were considered secondary to disease progression and one was reported due to ischemic cardiomyopathy and considered not related to drug. Two subjects had drug related deaths. One subject, treated at 10 mg/kg, had grade 4 pneumonitis and died with grade 5 sepsis. The other subject, treated at 1 mg/kg, developed grade 3 pneumonitis and grade 4 acute respiratory distress syndrome and died with grade 5 sepsis. Neither subject received steroids until the pulmonary symptoms were severe. Management algorithms, including the use of immunosuppressive agents such as corticosteroids and infliximab for treating pneumonitis and acute respiratory distress syndrome are known in the art.

Preliminary results demonstrated clinical activity in both of the trials mentioned above. Of the 39 subjects in CA2009001, three subjects had partial response (colorectal carcinoma, melanoma, and renal cell carcinoma) and ten subjects had stable disease. In CA209003, 91 subjects were evaluable for tumor response and complete or partial responses were reported at dose levels of 1, 3 and 10 mg/kg in subjects with non small cell lung cancer, renal cell carcinoma and melanoma. Data from these ongoing clinical trials were recently reported by Topalian SL, et al., New Eng J Med 2012; 366(26):2443-2454 (see also WO 2008/156712 (h409A11) and Hamid O et al., New Eng J Med 2013; 369:134-144).

### Example 4: Pharmacokinetics of Anti-PD-1 Antibody (nivolumab)

A single dose pharmacokinetic analysis of 39 subjects with cancer given nivolumab at 0.3, 1, 3 and 10 mg/kg revealed that the median Tₘₐₓ across single doses ranged from 1.6 to 3 hours with individual values ranging from 0.9 to 7 hours. The pharmacokinetics of nivolumab were linear in the range of 0.3 to 10 mg/kg with dose proportional increases in maximum serum concentration (Cₘₐₓ) and area under the concentration-time curve from time zero to infinity (AUC_{INF}), with low to moderate inter-subject variability observed at each dose level. The mean terminal elimination half-life of nivolumab was 17 to 25 days, which is consistent with the half-life of endogenous IgG4. Both the elimination and distribution of nivolumab were independent of the dose (data not shown).

### Example 5: Phase I Clinical Trial with IPH-2101

IPH-2101 (also known as 1-7F9 and described in WO 2006/003179) is a fully human anti-KIR monoclonal antibody that binds specifically, and with high affinity, to KIR2DL-1, 2 and 3 and KIR2DS-1 and 2, thus preventing interaction between KIR and HLA-C. A Phase I clinical trial with IPH-2101 in patients with AML has been completed. Single administration at doses of 0.0003, 0.003, 0.015, 0.075, 0.3, 1 and 3 mg/kg did not reach a maximally tolerated dose. Two Phase I studies and three Phase II studies are ongoing in patients with AML or multiple myeloma. In these studies, various dose levels were tested up to 3 mg/kg at an interval of every four weeks and the maximum number of cycles administered was six. Pharmacokinetic studies suggested a half life of 12-14 days at doses higher than 0.3 mg/kg. At a dose of 0.075 mg/kg, full KIR occupancy (>90%) was seen for less than 7 days. At a dose of 0.3 mg/kg, KIR occupancy decreased to less than 90% beginning on day 28. Sustained full KIR occupancy over four weeks was achieved at a dose of 3 mg/kg.

As of December 1, 2011, clinical safety data was available for 136 patients in these trials. Adverse events (AE) were reported in 128 of 136 (94%) subjects and included 183 of 734 (25%) reports that were possibly, probably, or definitely related to IPH-2101. AEs that were reported in more than one subject included general symptoms (chills, pyrexia, fatigue, weakness), gastrointestinal symptoms (nausea, vomiting, diarrhea), neurological symptoms (dizziness, headache, tremors), pulmonary symptoms (dyspnea), skin symptoms (erythema, pruritus, rash), others (flushing, hypertension, muscle spasms, myalgia), and laboratory abnormalities (hyperkalemia, increased lipase, decreased counts in leukocytes, neutrophils and platelets). These events were mostly Grade 1 and Grade 2 and tended to be more frequent at doses greater than 1 mg/kg. Only one patient with multiple myeloma experienced a serious adverse event (SAE), which was due to acute renal failure. Although deemed related to IPH-2101, the patient also had disease progression. Overall, IPH-2101 was tolerable at doses from 0.0003 to 3 mg/kg.

### Example 6: Pre-Clinical Pharamacology of Anti-KIR Antibody (lirilumab)

Lirilumab is a fully human, IgG4 monoclonal antibody that binds specifically and with high affinity to a subset of KIRs, namely KIR2DL-1, 2 and 3 and KIR2DS-1 and 2. Surface plasmon resonance analysis demonstrated that the mean monovalent affinity of lirilumab for recombinant soluble KIR2DL1 was 2.04 × 10⁻⁸ M (s.d. 0.31 × 10⁻⁸) and that for KIR2DL3 was 3.01 × 10⁻¹⁰ M (s.d. 0.41 × 10⁻¹⁰). The heavy and light chain amino acid sequences of LIRILUMAB are provided in SEQ ID NOs:1 and 2, respectively.

### Example 7: Lack of Toxicity of Anti-KIR Antibody (lirilumab) in Mice

Neither lirilumab nor IPH-2101 binds to NK cells from non-human primate or other species traditionally used for safety testing. However, Ly49C/I are murine inhibitory receptors that are functionally homologous to human KIR. There were no adverse findings in mice treated with lirilumab at 10 mg/kg once weekly for four weeks, or with the surrogate anti-Ly49 antibody 5E6 F(ab')2 twice weekly for 13 weeks (data not shown).

### Example 8: Clinical Pharmacology and Safety of Anti-KIR Antibody (lirilumab)

Safety data for the 136 subjects treated with IPH-2101 is described above in Example 5. Lirilumab comprises the same heavy and light chain variable regions as IPH-2101 (also known as 1-7F9), and thus binds to the same epitope as IPH-2101, but differs from IPH-2101 in that (1) it is prepared in Chinese hamster ovary (CHO) cells, whereas IPH-2101 is prepared from hybridoma cells, and (2) a stabilizing hinge mutation (S231P) has been introduced into lirilumab.

Preliminary pharmacodynamic assessment of KIR occupancy revealed that all three subjects who received 0.015 mg/kg of lirilumab had full saturation of KIR2D (>90% KIR occupancy) for less than 1 week. Subjects who received 0.3 mg/kg had full saturation for at least 8 weeks, which was prolonged even longer in those who received higher doses. Half of subjects (0.015, 0.3, 1 and 3 mg/kg), including all three in the last cohort tested, had modest transient increases in levels of interferon gamma (data not shown).

Additionally, a Phase I study involving a related antibody, IPH2101 (also designated 1-7F9 in WO 2006/003179), having identical variable regions to lirilumab, but lacking a stabilizing S241P hinge mutation) has been completed for subjects with advanced hematological malignancies (Vey N et al. (2012) Blood 120(22):4317-23). As of May 7, 2012, twenty subjects received IPH2101 at dose levels of 0.015, 0.3, 1, 3, 6, and 10 mg/kg. Six subjects had solid tumors (4 ovarian, 1 endometrial, 1 breast cancer) and 14 had hematological malignancies. The subjects in the lower three dose levels received four doses given at an interval of every four weeks. The subjects at the higher dose levels of 3, 6, and 10 mg/kg received one dose. There were no dose limiting toxicities. There was no trend in the frequency of AEs in relation to dose level. Eighteen of 20 (90%) subjects reported AEs. Most events were grade 1 (65%) or grade 2 (23%) in severity. Of a total of 111 AEs, 38 (34%) were considered related to lirilumab, the most common of which were fatigue (16%), headache (13%), pruritus (11%), asthenia (5%), constipation (5%), hypertension (5%), peripheral edema (5%) and rash (5%). There was only one grade 3 event that was related to lirilumab that occurred in a subject who received one dose at 6 mg/kg. This was an increase in lipase in a subject who entered the study with a grade 2 increase in lipase that returned to baseline 22 days later. There were no SAEs.

### Example 9: Pharmacokinetics of Anti-KIR Antibody (lirilumab)

Pharmacokinetic results from the on-going phase I study are pending. However, a PK model suggests that the PK profile of lirilumab is likely to be comparable to IPH-2101. In previous IPH-2101 phase I clinical trials in subjects with AML and multiple myeloma, a 2-compartment model with first order elimination was found to adequately describe the data with dose-dependent clearance, such that clearance decreased with increasing doses. The terminal half-life at the highest dose (3 mg/kg) was determined to be 18 days, which is consistent with reported values in the literature.

### Example 10: Inhibition of tumor growth in vivo by combination treatment with anti-KIR antibody and anti-PD-1 antibody

An experiment was conducted in a murine solid tumor model to test the hypothesis that the combination of anti-KIR and anti-PD-1 would potentiate anti-tumor efficacy. The rationale was to utilize pharmaceutical manipulation to coordinately regulate innate and adaptive immunity and recapitulate the biology seen in post-allogeneic transplant patients who have KIR mismatch. Both nivolumab (anti-human PD-1 antibody) and lirilumab (anti-human KIR antibody) recognize only human sequences. Thus, a murine specific PD-1 antibody, an anti-Ly49 antibody, and an F(ab)2 that recognizes Ly49C/I (which is the KIR homologue in mice) were used to test this hypothesis.

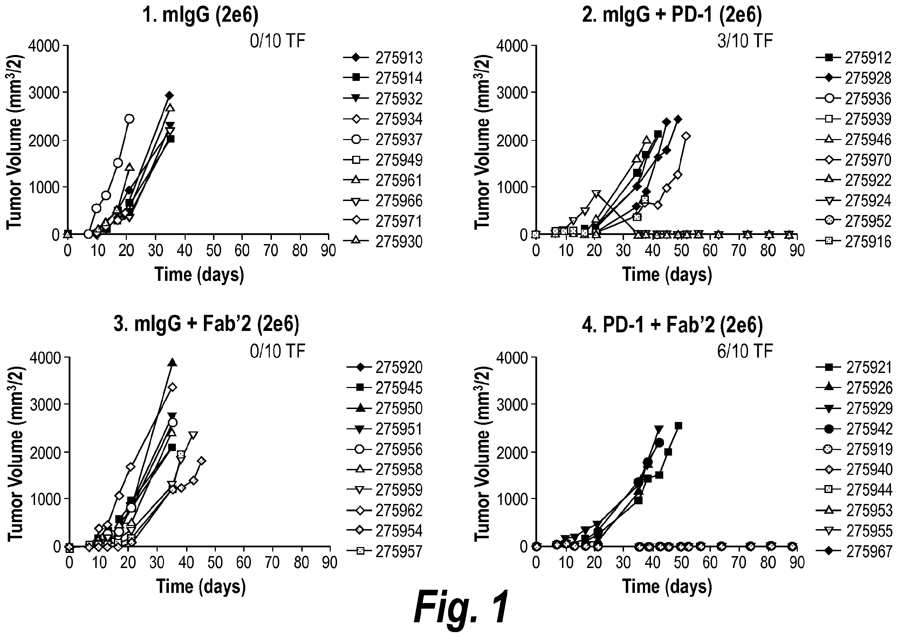

Mice were injected with the syngeneic MC38 murine colon carcinoma cell line and, following the formation of palpable tumors, were randomized to one of four cohorts to receive control IgG, anti-Ly49 antibody, anti-PD-1 antibody, or both antibodies. As shown in Figure 1, mice treated with a control IgG antibody had rapid growth of tumors (see upper left panel of Figure 1). Mice treated with anti-Ly49 antibody did not differ significantly from control animals (lower left panel of Figure 1). Those treated with a murine anti-PD-1 antibody showed latency in tumor progression and 30% of mice continued to be free of tumor (see upper right panel of Figure 1). Those treated with both anti-Ly49 and anti-PD-1 antibodies also had latency in tumor progression and 60% of mice had regression of established tumors (see lower right panel of Figures 1). These results provide pre-clinical evidence of the ability of an anti-KIR antibody to synergistically (i.e., more than additively) potentiate the efficacy of an anti-PD-1 antibody in a murine solid tumor model.

### Example 11: Phase 1 Trial in Patients Having Solid Tumors

A phase 1 trial of Anti-KIR Antibody (lirilumab) and Anti-PD-1 Antibody (nivolumab) is conducted in patients having advanced solid tumors to demonstrate the efficacy, including a synergistic effect, of administering lirilumab and nivolumab as a combination treatment (NCT01714739; Sanborn *et al.,* 2013).

### 1. Objectives

One objective of the study is to assess the safety and tolerability of lirilumab given in combination with nivolumab and to identify dose limiting toxicities (DLTs) and the maximally tolerated dose (MTD) of the combination, in subjects with advanced (metastatic and/or unresectable) solid tumors.

Other objectives include assessing the preliminary anti-tumor activity of the combination of lirilumab and nivolumab in subjects with advanced solid tumors, characterizing the pharmacokinetics (PK) of lirilumab and nivolumab when co-administered, monitoring immunogenicity of lirilumab and nivolumab administered as combination therapy, and assessing the pharmacodynamic effect in tumor tissue on tumor infiltrating lymphocyte (TIL) subsets from melanoma subjects treated with lirilumab given in combination with nivolumab.

Additional objectives include assessing the pharmacodynamic effects of lirilumab versus dose and/or exposure given in combination with nivolumab on biomarkers in peripheral blood, including NK cell and T cell compartments and serum proteins (cytokines and other immune modulators), assessing the pharmacodynamic activity in tumor tissue and peripheral blood in subjects treated with lirilumab and nivolumab who undergo optional biopsies, exploring potential associations between biomarker measures and anti-tumor activity, further characterizing KIR occupancy and NK function at multiple dose levels of lirilumab when given in combination with nivolumab, evaluating the potential association of subject KIR and HLA genotypes with clinical outcome, and assessing the landmark overall survival at three years following the start of therapy with the combination of lirilumab and nivolumab.

### 2. Study Design and Duration

The study is a phase I, open label study and is conducted in two parts. The first part of the study consists of a dose escalation assessment of the safety and tolerability of lirilumab administered with nivolumab in subjects with advanced solid tumors. The second part of the study includes 6 expansion cohorts of approximately 16 subjects each at either the maximally tolerated dose (MTD), maximally administered dose (MAD), or at an alternative dose. This part is disease restricted.

Subjects complete up to four periods of the study: Screening (up to 28 days), Treatment (up to a maximum of 2 years of study therapy), Clinical Follow-up (100 days), and Survival Follow-up (up to 3 years following the first dose of study drug). The Treatment Period consists of up to 12 eight-week treatment cycles. Each treatment cycle is comprised of 4 doses of nivolumab and 2 doses of lirilumab. nivolumab is administered on Days 1, 15, 29, and 43, and lirilumab is administered on Days 1 and 29 of each treatment cycle. On days where both study drugs are given, nivolumab is given first followed by lirilumab within 30 minutes of completing the 60 minute infusion of nivolumab. Following each treatment cycle, the decision to treat a subject with additional cycles of study therapy is based on tumor assessment (evaluation performed between Days 49 and 56 and completed before the first dose in the next cycle). Treatment decisions related to subject management are based exclusively on immune related (ir) response criteria, irRECIST (Wolchok JD, et al., Clin Cancer Res 2009; 15:7412-7420). Subjects with an overall response of irPD-unconfirmed, irSD, irPR, or irCR-unconfirmed at the end of a given cycle continue to the next treatment cycle. Subjects are generally allowed to continue study therapy until the first occurrence of any of the following: 1) achievement of irCR-confirmed; 2) completion of the maximum number of cycles, 3) have ir-PD confirmed, 4) clinical deterioration suggesting that no further benefit from treatment is likely, 5) intolerability to therapy; or 6) the subject meets criteria for discontinuation of study therapy. The subjects enter the Clinical Follow-up period, with visits scheduled on days 30, 60 and 100 to monitor for adverse events.

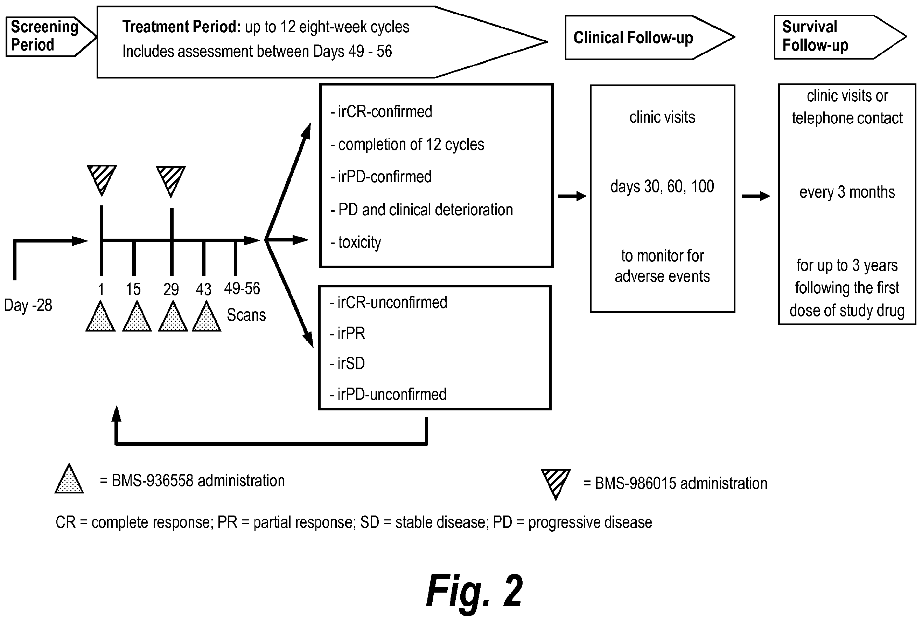

After completion of the Clinical Follow-up period, subjects enter the Survival Follow-up period. During this period, clinic visits or telephone contact every 3 months are performed to assess survival status. The duration of this period is up to 3 years following the first dose of study drug. A study schematic is shown in Figure 2.

Subjects who are in complete remission, but who progress during the Clinical Follow-up period or the Survival Follow-up period, are eligible to receive both study drugs, at the same doses and the same schedule that they received previously. Therapy continues until irCR-confirmed is attained, or for a period of one year. Subjects must again meet all of the eligibility criteria. Study drug is provided via an extension of the study, a rollover study requiring approval by responsible health authority and ethics committee, or through another mechanism.

The Screening Period lasts up to 28 days. The Treatment Period lasts up to 2 years. The Clinical Follow-up Period lasts 100 days. The Survival Follow-up Period lasts up to 3 years following the first dose of study drug. The total time on study for any individual subject does not exceed 3.1 years. The total duration of the study is 4.5 years from the time of the first visit of the first subject to the required survival follow-up of the last subject enrolled.

### 3. Dose Escalation

A 6+3 design is used to assess the safety of lirilumab given in combination with nivolumab. The dosages during dose escalation are provided below in Table 1.

**Table 1: Dosages during Dose Escalation**

| **Dose Level** | **Total Subjects** | **lirilumab (IV; mg/kg)** | **nivolumab (IV; mg/kg)** |
|---|---|---|---|
| 1 | n = approximately 6-12 | 0.1 | 3 |
| 2 | n = approximately 6-12 | 0.3 | 3 |
| 3 | n = approximately 6-12 | 1 | 3 |
| 4 | n = approximately 6-12 | 3 | 3 |
| Total | n = approximately 24-48 | | |

The Dose Limiting Toxicity (DLT) observation period lasts for 8 weeks (Cycle 1). Six subjects are treated at each dose level with expansion up to 9 subjects if two dose limiting toxicities are observed in the first 6 subjects. If 0 or 1 DLTs occur in a cohort of 6 subjects, a new cohort of 6 subjects is treated at the next higher dose level. If 2 of 6 DLTs occur, that cohort is expanded to 9 subjects. If 3 or more of 6, or 3 or more of 9 subjects experience DLTs within a cohort, then that dose level is determined to have exceeded the maximum tolerated dose (MTD). If no MTD is reached through cohort 4, then additional cohorts at 6 mg/kg lirilumab and 10 mg/kg BMS 986015, given in combination with 3 mg/kg nivolumab are considered based on the aggregate safety experience during dose escalation .

To further explore emerging safety signals during dose escalation, a total of up to 12 subjects are accrued to any dose level. The additional enrollment is only permitted once the dose level in the cohort has been evaluated and declared safe for dose escalation. Only DLTs in the initial 6-9 subjects enrolled in a dose level are formally evaluated in dose escalation and subsequent determination of the MTD. However, safety data from all treated subjects is considered in dose selection for cohort expansion.

No intra-subject dose escalation or reduction is allowed. Subjects who withdraw from the study during the DLT period for reasons other than a DLT are replaced within the same dose level. For the purpose of making decisions on dose escalation from a safety perspective, subjects are considered evaluable if they have received 3 out of the 4 scheduled nivolumab doses through the 8 week observation period, only if the one missed dose was secondary to non-medical reasons.

Dose escalation is based on the number of dose limiting toxicities (DLTs) experienced during Cycle 1. The initial 6 subjects at each dose level have peripheral blood evaluation for PD markers.

All available clinical and laboratory data, and the nature, time of onset and time to resolution of DLTs observed during dose escalation are reviewed to determine whether an alternative dose schedule should be examined, if needed. If agreed upon, the alternative schedule is identified by a protocol amendment.

### 4. Cohort Expansion

The purpose of the cohort expansions is to gather additional safety, tolerability, preliminary efficacy and pharmacodynamic information regarding the combination of lirilumab and nivolumab. Once the safety profile of all doses tested are characterized and the MTD of combined administration of lirilumab and nivolumab has been defined, the cohort expansion is initiated at the MTD, the maximum administered dose (MAD), or an alternate dose. Six expansion cohorts are restricted to the tumor types listed below in Table 2.

**Table 2: Tumor Types Eligible For Cohort Expansion**

| **Tumor Type** | **Total Subjects** |
|---|---|
| Non small cell lung cancer - squamous histology | approximately 16 |
| Non small cell lung cancer - non-squamous histology | approximately 16 |
| Renal Cell Carcinoma with a clear cell component | approximately 16 |
| Melanoma | approximately 16 |
| Colorectal Cancer | approximately 16 |
| Serous Ovarian Carcinoma | approximately 16 |
| Total | approximately 96 |

Continuous evaluation of toxicity events in the cohort expansions is performed throughout enrollment in the expansion cohorts. If the rate of DLTs exceeds 33%, the findings are discussed and further enrollment is interrupted. If an expansion cohort is discontinued due to toxicity, a new cohort is initiated at a previously tested lower dose level.

Continuous evaluation of toxicity events in the cohort expansions is performed throughout enrollment in the expansion cohorts. If the rate of DLTs exceeds 33%, the findings are discussed and further enrollment is interrupted. If an expansion cohort is discontinued due to toxicity, a new cohort is initiated at a previously tested lower dose level.

### 5. Treatments

The study treatments include nivolumab and lirilumab. Table 1 indicates the dose level to be used for each panel. Expansion cohorts are treated at the highest tested dose or a different dose level as selected by the sponsor. For treatment visits where both lirilumab and nivolumab are administered, nivolumab is administered first followed by lirilumab within 30 minutes after completion of the nivolumab infusion.

### 6. Dose Limiting Toxicities

lirilumab has the potential to augment the frequency and severity of previously described adverse events associated with nivolumab, or to develop new toxicities. Dose limiting toxicity (DLT) is determined based on the incidence, intensity and duration of adverse events that are related to study drug, and that occur within 56 days (8 weeks, through the completion of Cycle 1) of initiation of study drug. The severity of adverse events is graded according to the NCI CTCAEv4. Hepatic, non-hematologic, and hematologic DLT are defined separately as outlined below

Any of the following events are considered a hepatic DLT:
- ALT or AST > 8X ULN, regardless of duration
- ALT or AST > 5X and ≤ 8X ULN, that fails to return to Grade 1 or less within 5 days despite medical intervention
- Grade 3 total bilirubin
- ALT or AST > 3X ULN and concurrent total bilirubin > 2X ULN

Any of the following events are considered a Non-Hematologic DLT:
- Grade 2 eye pain or reduction in visual acuity that requires systemic treatment
- Grade 2 eye pain or reduction in visual acuity that does not respond to topical therapy and that does not improve to Grade 1 within 2 weeks of initiation of topical therapy
- Grade 3 Non-Hepatic or Non-Hematologic toxicity, with the following exceptions:

The Following Grade 3 Non-Hematologic events are not be considered DLTs:
- Grade 3 electrolyte abnormality that lasts less than 72 hours, is not clinically complicated, and resolves spontaneously or responds to conventional medical intervention
- Grade 3 increase in amylase or lipase that is not associated with clinical or radiographic evidence of pancreatitis
- Grade 3 nausea or vomiting that lasts less than 48 hours, and resolves to Grade 1 or less either spontaneously or with conventional medical intervention
- Grade 3 fever that lasts less than 72 hours, and is not associated with hemodynamic compromise (including hypotension, or clinical or laboratory evidence of end organ perfusion impairment)
- Grade 3 endocrinopathy that is well controlled by hormone replacement
- Grade 3 tumor flare (defined as pain, irritation or rash that localizes to sites of known or suspected tumor)
- Grade 3 fatigue
- Grade 3 infusion reaction that returns to Grade 1 in less than 6 hours

Any of the following events are considered a Hematologic DLT:
- Grade 4 neutropenia that lasts longer than 5 days
- Grade 4 thrombocytopenia
- Grade 3 thrombocytopenia associated with clinically significant bleeding
- Grade 3 febrile neutropenia that lasts longer than 48 hours
- Grade 3 hemolysis

### 7. Guidelines for Dose Modification

Intrasubject dose escalation or reduction of lirilumab and BMS-986558 is not permitted in this study in order to allow better evaluation of extended safety and efficacy at individual dose levels.

Subjects who experience a DLT must have therapy held, pending resolution of the toxicity. If the adverse event resolves to grade 1 or less, or to baseline, in severity within 28 days, then therapy resumes at the same doses for both study drugs. If the toxicity resolves after 28 days, and the investigator believes that the subject is deriving clinical benefit, then the subject is eligible to resume the study drugs. If the subject then experiences a subsequent DLT, that also resolves and the investigator continues to believe that the subject is deriving clinical benefit, then the subject is eligible to resume the study drugs.

Subjects are required to permanently discontinue both study drugs for the following:
- Any grade 4 adverse event, with the exception of: grade 4 electrolyte abnormalities that resolves ≤ 72 hours, grade 4 neutropenia ≤ 5 days in duration, or grade 4 lymphopenia ≤ 5 days in duration.

Any adverse event with clinical risk is assessed on a case by case basis to determine the risks and benefits of continuing on therapy following resolution versus discontinuing therapy permanently. High grade events involving the central nervous system, eyes, liver or lung, would normally require permanent discontinuation, unless there are elements of the individual's history and clinical course that suggests a higher likelihood of benefit over risk with continued therapy upon resolution.

### 8. Safety Assessments

Adverse events are assessed continuously during the study and for 100 days after the last treatment. Adverse events are coded using the most current version of MedDRA and reviewed for potential significance and importance. Adverse events are evaluated according to the NCI CTCAE Version 4.0. Subjects should be followed until all treatment related adverse events have recovered to baseline or are deemed irreversible by the investigator.

### 9. Efficacy Assessments

Disease assessment with computed tomography (CT) and/or magnetic resonance imaging (MRI), as appropriate, are performed at baseline and every 8 weeks until confirmed disease progression, at the completion of follow-up, or until subjects withdraw from the study. Disease assessments at other timepoints are performed if the investigator is concerned about tumor progression. Tumor responses are determined for appropriate populations of subjects as defined by RECIST v1.1 (Eisenhauer EA, Eur J Cancer 2009; 45:228-247), as well as by immune-related response criteria, irRECIST (Wolchok JD, et al., Clin Cancer Res 2009; 15:7412-7420). Treatment decisions related to subject management are based exclusively on irRECIST criteria. Scans and measurements are collected centrally to be reviewed by independent radiologists using irRECIST and/or RECIST v1.1 criteria at a later date, or at any time during the study.

Changes in tumor measurements and tumor responses are assessed by the investigator using irRECIST criteria. Investigators also report the number and size of new lesions that appear while on-study. The timepoint tumor assessments are reported on the CRF based on investigators' assessment using irRECIST criteria. In addition RECIST v1.1 timepoint assessments are derived programmatically.

### 10. Exploratory Efficacy Assessments

Overall survival data is collected up to 3 years from the start of study drug treatment. Serum samples for lirilumab and/or nivolumab PK assessments are collected for all subjects in both dose escalation and cohort expansion. Pharmacokinetics of lirilumab are derived from serum concentration versus time. The assessed pharmacokinetic parameters include:
- Cmax: Maximum observed serum concentration
- Tmax: Time of maximum observed serum concentration
- AUC(0-T): Area under the plasma concentration-time curve from time zero to time of last quantifiable concentration
- AUC(INF): Area under the plasma concentration-time curve from time zero extrapolated to infinite time
- Ctrough: Trough observed serum concentration
- AUC(TAU): Area under the concentration-time curve in one dosing interval
- CL: Clearance
- Vss: Volume of distribution at steady state
- t_{1/2}: Half-life

Individual subject pharmacokinetic parameter values are derived by non compartmental methods by a validated pharmacokinetic analysis program. Actual times are used for the analyses. In addition, nivolumab end of infusion and trough (Cmin) concentrations are calculated at specified visits.

Serum samples are analyzed for lirilumab and nivolumab by a validated immunoassay. Additionally, samples are banked for potential exploratory pharmacokinetic analysis by an orthogonal bioanalytical method (e.g., LC/MS-MS).

### 11. Exploratory Biomarker Assessments

The pharmacodynamics of lirilumab and nivolumab in combination are assessed by quantifying biomarkers from peripheral blood.

### 12. Assessments for Patients during Dose Escalation

NK Cell and T Cell Functional Assessment and KIR Occupancy: Pre-treatment and on-treatment PBMCs are used to investigate the relationship between KIR occupancy (target of lirilumab) and NK cell function as measured by CD107a and intracellular INFγ expression using flow cytometry in a co-culture assay with surrogate target cells. Specifically, NK cells are isolated from PBMCs and are co-cultured with target cells (HLA class I-positive and HLA-class I negative) in the presence of excess lirilumab to assess the induction of NK cytolytic activity from KIR-positive cells as a function of dose, time after dose, degree of KIR occupancy, and circulating levels of lirilumab (PK). Understanding the relationship between NK cell function with KIR occupancy or circulating lirilumab levels is important in establishing optimal drug dosage and/or timing of evaluating other biomarkers. PBMCs are also used to investigate the effects of lirilumab and nivolumab on T cell function as measured by intracellular INFγ expression using flow cytometry. Specifically, T cell subsets are incubated in anti-CD3-coated plates to assess T cell activation as a function of dose, time after dose, and circulating levels of lirilumab and nivolumab (PK). Understanding the relationship between T cell activation and various dose combinations of lirilumab and nivolumab levels is important in establishing optimal drug dosage and/or timing of evaluating other biomarkers. These studies are performed in the first six subjects in each dose cohort.

Immunophenotyping of NK Cell and T Cell Subsets: The relative proportion of lymphocyte subsets is assessed from peripheral blood samples. Additionally, PBMCs are used to characterize and quantify specific markers of inhibition and activation on NK cell and T cell subsets by polychromatic flow cytometry. Immunophenotyping of Treg cells includes, but is not limited to: HLA-DR, CD3, CD4, FoxP3, PD-L1, PD-1, LAG-3, ICOS, and CD25. Immunophenotyping of memory/effector T cells includes, but is not limited to: CCR7, CD45RA, CD27, CD28, CD3, CD4, CD8, Ki67, HLA-DR, PD-L1, PD-1, CTLA4, and ICOS. NK cell immunophenotyping includes, but is not limited to: CD56, CD3, CD16, CD54, CD94, KIR, NKG2D, NKp30, NKp46, IL-21R, Ki67, CD25, and granzyme B.

Immune Modulation Analysis of Soluble Factors: Pre-treatment and on-treatment serum levels of chemokines, cytokines and other immune mediators are assessed by techniques that include, but are not limited to ELISA or multiplex assays. Analytes include markers of immune activation, modulation, or inflammation such as IFN-γ, soluble NKG2D ligands (i.e., soluble MICA), and sCD25.

Expression of KIR on NK Cells: An absolute enumeration of KIR-positive expressing cells is determined from peripheral blood samples collected pre-treatment and on-treatment. Flow cytometry is used to assess not only the percent positive KIR-expressing cells (KIR2DL1/2/3) but also to quantitate the amount of KIR expression.

### 13. Assessments for Patients during Cohort Expansion

Blood samples are obtained from all subjects in cohort expansion pre-treatment to isolate DNA for the determination of KIR and HLA genotypes. Polymerase chain reaction (PCR) is used to define the genotypes that will then be correlated with clinical outcome following lirilumab in combination with nivolumab.

### 14. Assessments for Subjects Undergoing Tumor Biopsies

Blood is obtained for all subjects who consent to tumor biopsies in order to obtain tumor/normal pairs. Tumor biopsies are obtained pre-treatment and on-treatment (at the end of week 16) in a minimum of ten subjects in the melanoma cohort expansion. For any reason, if a subject cannot undergo the on-treatment biopsy, the first sample is not included as part of the requirement for 10 subjects with paired pre-treatment and on-treatment samples. Subjects are offered the opportunity to undergo post-treatment biopsy when possible. All other subjects are also offered the opportunity of undergoing tumor biopsies. Tumor samples are used to evaluate specific tumor infiltrating lymphocyte populations (NK cells, Treg cells, CTLs) present prior to, during, and possibly after therapy to assess the potential mechanism of action and as a potential biomarker of response. Tumor associated lymphocyte expression of KIR is also being explored on tumor specimens. In addition, tumor-expressed proteins (i.e. PD-L1 and HLA class I) are evaluated by IHC to determine possible associations with clinical response or pharmacodynamic effects to the combination of lirilumab and nivolumab. If there is acceptable quantity of tissue collected, sections from tumor biopsies collected at pre-treatment and on-treatment are cryopreserved for potential future gene expression analyses. Genes of interest include but are not limited to PD-1, PD-L1, KIR, and LAG-3. Simultaneous collections of peripheral blood/serum samples and tumor tissue (although limited in number of tumor biopsies) from the same subject are required to help understand and correlate pharmacodynamic events resulting from combined blockade of KIR and PD-1 and inform potential mechanisms or clinical outcome.

A minimum of 10 subjects in the melanoma expansion cohort must have at least one lesion large enough to undergo repeated biopsies (pre-treatment, on-treatment, and possibly post-treatment biopsies) via core needle (minimum size 16 gauge) or have at least two distinct lesions eligible for core needle or excisional biopsies. These lesions must not be the subject's only target lesions or sites that have received prior radiation therapy. Subjects in all other expansion cohorts are given the opportunity to undergo biopsies if deemed of acceptable clinical risk. The core needle length is greater than 5 mm. At least two core biopsies should be taken at each time point; but collection of additional cores is strongly encouraged, if deemed clinically safe by the investigator. Punch and excisional biopsies are also acceptable. The ideal minimal tumor volume is 150 mm³. Pathologic confirmation is strongly encouraged at the time of tumor biopsy to confirm adequate tissue collection and biopsy quality. All biopsies collected must have a detailed pathology report submitted with the specimen. Detailed instructions of the obtaining, processing, labeling, handling, storage and shipment of these specimens are provided in a separate Procedure Manual at the time of study initiation. Subjects whose screening biopsy yields inadequate tissue quantity or quality are allowed to continue in the study. These subjects are replaced in order to obtain 10 subjects with pre-treatment biopsies. If subjects have a response to treatment, on-treatment and post-treatment biopsies are not possible.

### 15. Immunogenicity Assessments

Serum samples for analysis of development of ADA are drawn in conjunction with analysis of lirilumab and nivolumab serum concentrations and are collected from all subjects pre-dose on Day 1, 15, and 29 of Cycle 1, Day 29 of Cycle 2, Day 1 of Cycle 3, end of treatment, and all 3 clinical follow-up visits. These serum samples are analyzed for ADA by a validated immunoassay. Additionally, lirilumab and nivolumab samples are banked for potential exploratory immunogenicity analysis by an orthogonal bioanalytical method (e.g., analysis of drug-ADA immune complexes).

### 16. Adverse Events

An adverse event (AE) is defined as any new untoward medical occurrence or worsening of a preexisting medical condition in a clinical investigation subject administered an investigational (medicinal) product and that does not necessarily have a causal relationship with this treatment. An AE can therefore be any unfavorable and unintended sign (such as an abnormal laboratory finding), symptom, or disease temporally associated with the use of investigational product, whether or not considered related to the investigational product.

The causal relationship to study drug is determined by a physician and should be used to assess all adverse events (AE). The casual relationship can be one of the following:
Related: There is a reasonable causal relationship between study drug administration and the AE.
Not related: There is not a reasonable causal relationship between study drug administration and the AE.

The term "reasonable causal relationship" means there is evidence to suggest a causal relationship.

Adverse events can be spontaneously reported or elicited during open-ended questioning, examination, or evaluation of a subject. (In order to prevent reporting bias, subjects should not be questioned regarding the specific occurrence of one or more AEs.)

A serious adverse event (SAE) is any untoward medical occurrence that at any dose:
- results in death
- is life-threatening (defined as an event in which the subject was at risk of death at the time of the event; it does not refer to an event which hypothetically might have caused death if it were more severe)
- requires inpatient hospitalization or causes prolongation of existing hospitalization
- results in persistent or significant disability/incapacity
- is a congenital anomaly/birth defect
- is an important medical event (defined as a medical event(s) that is not immediately life-threatening or result in death or hospitalization but, based upon appropriate medical and scientific judgment, jeopardizes the subject or requires intervention [e.g., medical, surgical] to prevent one of the other serious outcomes listed in the definition above).

Examples of such events include, but are not limited to, intensive treatment in an emergency room or at home for allergic bronchospasm; blood dyscrasias or convulsions that do not result in hospitalization. Potential drug induced liver injury (DILI) is also considered an important medical event.

Suspected transmission of an infectious agent (e.g., pathogenic or nonpathogenic) via the study drug is an SAE. Although pregnancy, overdose, cancer, and potential drug induced liver injury (DILI) are not always serious by regulatory definition, these events are handled as SAEs. Any component of a study endpoint that is considered related to study therapy (e.g., death is an endpoint, if death occurred due to anaphylaxis, anaphylaxis is reported) should be reported as SAE.

The following hospitalizations are not considered SAEs:
- a visit to the emergency room or other hospital department < 24 hours, that does not result in admission (unless considered an important medical or life-threatening event)
- elective surgery, planned prior to signing consent
- admissions as per protocol for a planned medical/surgical procedure
- routine health assessment requiring admission for baseline/trending of health status (e.g., routine colonoscopy)
- medical/surgical admission other than remedying ill health state and planned prior to entry into the study. Appropriate documentation is required in these cases
- admission encountered for another life circumstance that carries no bearing on health status and requires no medical/surgical intervention (e.g., lack of housing, economic inadequacy, care-giver respite, family circumstances, administrative).

Following the subject's written consent to participate in the study, all SAEs, whether related or not related to study drug, are collected, including those thought to be associated with protocol-specified procedures. All SAEs are collected that occur during the screening period and within 100 days of discontinuation of dosing. If applicable, SAEs are collected that relate to any later protocol-specified procedure (e.g., a follow-up skin biopsy). The investigator should report any SAE occurring after these time periods that is believed to be related to study drug or protocol-specified procedure. An SAE report should be completed for any event where doubt exists regarding its status of seriousness. If the investigator believes that an SAE is not related to study drug, but is potentially related to the conditions of the study (such as withdrawal of previous therapy, or a complication of a study procedure), the relationship should be specified in the narrative section of the SAE Report Form. SAEs, whether related or not related to study drug, and pregnancies are reported within 24 hours.

The collection of nonserious AE information should begin at initiation of study drug. Nonserious AE information should also be collected from the start of a placebo lead-in period or other observational period intended to establish a baseline status for the subjects.

Nonserious AEs should be followed to resolution or stabilization, or reported as SAEs if they become serious. Follow-up is also required for nonserious AEs that cause interruption or discontinuation of study drug and for those that are present at the end of study treatment as appropriate. All identified nonserious AEs are recorded and described on the nonserious AE page of the CRF (paper or electronic). Completion of supplemental CRFs is requested for AEs and/or laboratory abnormalities that are reported/identified during the course of the study.

### 17. Statistical considerations

Dose Escalation: As this is a Phase 1 dose escalation trial, the sample size at each dose cannot be determined exactly, as it depends on the number of observed toxicities. Between 6 and 9 subjects approximately are treated during dose escalation in each dose level, and up to 12 subjects are dosed at selected dose levels. Using a 6+3 design ensures 6 subjects at each dose to assess a signal on potential pharmacodynamic effects of the studied biomarkers.

Cohort Expansion: During cohort expansion, approximately 16 subjects are enrolled in each of 6 tumor types and treated at the previously determined MTD, MAD, or at an alternative dose. In an expansion cohort, if 2 (12.5%), 3 (18.8%), or 4 (25%) responses are observed, then the lower limits of the 90% one-sided confidence intervals for the objective response rate are 3.4%, 7.1% and 11.4%, respectively. In addition, 4 responses would need to be observed in 16 subjects so that the 80% confidence interval is entirely above 11% for the response rate. These calculations are based on the Clopper-Pearson method for exact confidence intervals. In addition, if the true objective response rate (ORR) in a tumor type/expansion cohort is 15%, then with 16 patients in each cohort there is 72% chance of observing at least 2 responses, and 44% chance of observing at least 3 responses, and there is 28% chance of observing 0 or 1 response (false negative rate). If the true ORR in a tumor type is 5% rather than 15%, then there is 19% and 4% chance respectively that there are at least 2 or at least 3 responses in 16 subjects (false positive rate).

Populations for Analyses:
- All Enrolled Data set: subjects who signed informed consent and registered in the study.
- All Treated Data set: all subjects who received at least one dose of either study drug.
- Response-Evaluable Data Set: all treated subjects who receive either study drug, have a baseline tumor assessment with measurable disease, and one of the following:
   - at least one evaluable on-treatment tumor assessment,
   - clinical progression, or
   - death prior to the first on-treatment tumor evaluation.
- lirilumab Pharmacokinetic Data Set: all subjects who receive at least one dose of lirilumab and have adequate serum concentration data for lirilumab PK.
- nivolumab Pharmacokinetic Data Set: all subjects who receive at least one dose of nivolumab and have adequate nivolumab PK.
- lirilumab Immunogenicity Data Set: all subjects who receive at least one dose of lirilumab and have at least one ADA sample available.
- nivolumab Immunogenicity Data Set: all subjects who receive at least one dose of nivolumab and have at least one ADA sample available.
- Biomarker Data Set: all treated subjects who have biomarker data available.

Endpoint Definitions: Safety is the primary endpoint in this Phase 1 study. All subjects who receive at least one dose of lirilumab or nivolumab are evaluated for safety as measured by the occurrence of adverse events, serious adverse events, deaths and laboratory abnormalities, assessed during treatment and for 100 days in follow-up.

The primary objective (to assess the safety and tolerability of lirilumab given in combination with nivolumab and to identify dose limiting toxicities (DLTs) and the maximally tolerated dose (MTD) of the combination) is measured by the following primary endpoints.
a) Incidence of adverse events: all non-serious adverse events are collected from Day 1 until 100 days after the subject's last dose of study drug or until they discontinue the study. All serious adverse events are collected from the date of the subject's written consent until 100 days after discontinuation of dosing or until they discontinue the study.
b) Incidence of clinical laboratory test abnormalities including hematology and serum chemistry, and thyroid panel abnormalities, assessed at specified time points.

Assessments are based on adverse event reports and the results of vital sign measurements, electrocardiograms (ECGs), physical examinations, imaging studies, and clinical laboratory tests. Adverse events are categorized using the most current version of the Medical Dictionary for Regulatory Activities (MedDRA); both AEs and laboratory tests are graded using National Cancer Institute (NCI) Common Terminology Criteria for Adverse Events (CTCAE) v4. All subjects who receive study drug therapy are evaluated for safety as measured by the rate of adverse events (AEs), and serious adverse events (SAEs), and are assessed during treatment and for 100 days in follow-up.

The secondary objective of assessing preliminary anti-tumor activity is based on endpoints described using irRECIST (Wolchok JD, et al., Clin Cancer Res 2009; 15:7412-7420) and RECIST v1.1 (Eisenhauer EA, Eur J Cancer 2009; 45:228-247). For the purposes of patient management, clinical decision making is based exclusively on irRECIST. Therefore timepoint tumor response evaluations are recorded on the CRF based on investigators' assessments using irRECIST criteria. Statistical analysis and reporting is based on both criteria.

Best overall response (BOR) is the best response designation recorded from the start of the study treatment until the end of treatment taking into account any requirement for confirmation, based on RECIST v1.1 or irRECIST criteria. CR or PR determinations included in the BOR assessment are confirmed by a consecutive second (confirmatory) evaluation meeting the criteria for response that is performed at least 4 weeks after the criteria for response are first met. The above is determined based on tumor measurements occurring every 8 weeks during the Treatment period (Cycle 1 Day 1 through Cycle 12 Day 56), and once during the Clinical Follow-up period.

Study level endpoints used to assess this objective are defined as follows:
Objective response rate (ORR) is defined as the total number of subjects whose BOR is either CR or PR divided by the total number of subjects in the population of interest.

Duration of Response (DOR) computed only for subjects with a BOR of CR or PR is defined as the number of days between the date of first response and the subsequent date of objectively documented disease progression based on the criteria (RECIST v1.1 or irRECIST) or death, whichever occurs first. For those subjects who remain alive and have not progressed or received subsequent therapy, duration of response is censored on the date of last tumor assessment. Subjects who receive subsequent therapy are censored at the start of subsequent therapy.

Progression-Free Survival Rate (PFSR) is defined as the probability of a subject remaining progression-free and surviving to 24 weeks. The probability is computed based on the number of days between the first dose of study drug and progressive disease or death, as defined by each criterion. For those subjects who remain alive and have not progressed, PFS is censored on the date of the last tumor assessment. The above is calculated based on tumor measurements occurring every 8 weeks during treatment and at planned timepoints during the Clinical Follow-up period.

Pharmacokinetics (PK): lirilumab maximum concentration Cmax,(µg/mL), time to maximum concentration Tmax (hr), Area under the curve AUCTAU (µg.hr/mL), Area under the curve AUCinf (µg.hr/mL), Clearance (L/day), Volume of distribution (Vss), half-life (t1/2), and trough concentration Cmin (µg/mL) is evaluated using non-compartmental analysis in all study subjects. In addition, nivolumab end of infusion and trough (Cmin) concentrations are calculated at specified visit.

Immunogenicity: Occurrence of specific anti-drug antibodies to lirilumab and nivolumab is determined from measurements on weeks 1, 3, 5, 13, 17, end of treatment, and all 3 clinical follow-up visits.

Biomarkers: Measures of TILs, PD-L1 and HLA Class I expression using immunohistochemistry on mandatory tumor biopsies from a minimum of ten melanoma cohort expansion subjects, including baseline and changes from baseline outcomes.

Exploratory Endpoint(s): Biomarkers from peripheral blood will include measures of KIR and HLA genotypes, KIR occupancy, NK and T cell functional assays, soluble factors, KIR expression on NK cells. Overall Survival (OS) is an exploratory efficacy endpoint.

### 18. Analyses

Demographics and Baseline Characteristics: Frequency distributions of gender and race are tabulated. Summary statistics for age, body weight, and height are collected, and Body Mass Index (BMI) is derived.

Efficacy Analyses: Individual best overall response (BOR), duration of response and PFS is listed using RECIST v1.1 and irRECIST criteria. BOR outcomes are tabulated by disease type and dose. The objective response rate (ORR) and PFS rate (e.g. at 24 weeks) and corresponding confidence interval are provided by tumor type and treatment. The duration of response, duration of stable disease and PFS are estimated by Kaplan-Meier methodology by disease type, depending on data availability. PFS rates at 24 weeks are similarly estimated, based on K-M methodology. ORR, duration of response and PFS analyses will include subjects in the cohort expansion phase and subjects in dose escalation matching those in cohort expansion by disease type and treatment. Individual changes in the tumor burden over time are presented graphically within a disease type. Landmark overall survival is assessed as part of exploratory efficacy analysis, by Kaplan-Meier plots and medians for each tumor type.

Safety Analyses: All recorded adverse events are listed and tabulated by system organ class, preferred term and treatment. Vital signs and clinical laboratory test results are listed and summarized by treatment. Any significant physical examination findings, and clinical laboratory results are listed. ECG readings are evaluated by the investigator and abnormalities, if present, are listed.

Pharmacokinetic Analyses: Summary statistics are tabulated for the pharmacokinetic parameters of lirilumab by dose and study day/week. To describe the dependency on dose of anti-KIR, scatter plots of Cmax and AUC(TAU) versus dose are provided for each day measured. Dose proportionality of lirilumab when co-administered with nivolumab are assessed based on a power model. nivolumab end of infusion and trough (Cmin) concentration are tabulated by summary statistics. This data is also pooled with other datasets for population PK analysis which are part of a separate report.

Biomarker Analyses: The pharmacodynamic effect of lirilumab on Tumor Infiltrating Lymphocytes (TILs) and expression of tumor markers including PD-L1 and HLA Class I are assessed by summary statistics, and investigated graphically to explore patterns of change, e.g., with drug exposure, for subjects in the melanoma expansion cohort. In addition, the correlation of TIL changes and tumor marker expression with measures of peripheral blood markers are explored graphically, or by appropriate statistical methods based on data availability, for assessing associations.

Exploratory Biomarker Analyses: The pharmacodynamic effect of lirilumab on KIR occupancy and the combination of nivolumab given with lirilumab on markers in peripheral blood and serum proteins are assessed by summary statistics, and investigated graphically to explore patterns of change over time, and how the patterns differ among dose levels and exposure. If there is a meaningful indication in the pattern over time, further analysis (e.g, by linear mixed model) is performed to characterize the relationship. Pharmacodynamic effects on tumor markers in cohorts other than melanoma are similarly assessed depending on data availability. Associations between biomarker measures from peripheral blood or tumor biopsy and clinical outcomes are also explored graphically, and further assessed as needed by methods such as, but not limited to, logistic regression, and characterized by appropriate statistics.

Other Analyses: A listing is provided of all available immunogenicity data. Additionally, a listing of immunogenicity data from those subjects with at least one positive anti-drug antibody (ADA) at any time point is provided by treatment for each analyte. The frequency of subjects with at least one positive ADA assessment, and frequency of subjects who develop ADA after a negative baseline assessment are provided. To examine the potential relationship between immunogenicity and safety, the frequency and type of AEs of special interest are examined by overall immunogenicity status. Associations between trough concentrations of lirilumab (or nivolumab) and corresponding ADA assessments are explored.

Interim Analyses: Data emerging from this study is needed for timely decisions about adjustments to procedures in subsequent parts of the study. Therefore, data is reviewed prior to the final lock of the study database. Additional interim analyses are also performed for administrative purposes or publications. Analyses only consist of listings, summaries, and graphs of the available data. No formal inferences requiring any adjustment to statistical significance level are performed. Efficacy analyses based on interim data use response evaluable or all treated populations depending on the purpose of the analysis.

**SEQUENCE SUMMARY**

| **SEQ ID NO:** | **SEQUENCE** |
|---|---|
| 1 | Heavy Chain Amino Acid Sequence Anti-KIR mAb (IPH2102 / lirilumab) (CDRs underlined) |
| | QVQLVQSGAE VKKPGSSVKV SCKASGGTFS FYAISWVRQA PGQGLEWMGG FIPIFGAANY AQKFQGRVTI TADESTSTAY MELSSLRSDD TAVYYCARIP SGSYYYDYDM DVWGQGTTVT VSSASTKGPS VFPLAPCSRS TSESTAALGC LVKDYFPEPV TVSWNSGALT SGVHTFPAVL QSSGLYSLSS VVTVPSSSLG TKTYTCNVDH KPSNTKVDKR VESKYGPPCP PCPAPEFLGG PSVFLFPPKP KDTLMISRTP EVTCVVVDVS QEDPEVQFNW YVDGVEVHNA KTKPREEQFN STYRVVSVLT VLHQDWLNGK EYKCKVSNKG LPSSIEKTIS KAKGQPREPQ VYTLPPSQEE MTKNQVSLTC LVKGFYPSDI AVEWESNGQP ENNYKTTPPV LDSDGSFFLY SRLTVDKSRW QEGNVFSCSV MHEALHNHYT QKSLSLSLGK |
| 2 | Light Chain Amino Acid Sequence Anti-KIR mAb (IPH2102 / lirilumab) (CDRs underlined) |
| | EIVLTQSPVT LSLSPGERAT LSCRASQSVS SYLAWYQQKP GQAPRLLIYD ASNRATGIPA RFSGSGSGTD FTLTISSLEP EDFAVYYCQQ RSNWMYTFGQ GTKLEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| 3 | Heavy Chain Variable Region (VH) Amino Acid Sequence Anti-KIR mAb (IPH2102 / lirilumab) - (SEQ ID NO:17 from WO 2006/003179) |
| | QVQLVQSGAEVKKPGSSVKVSCKASGGTFSFYAISWVRQAPGQGLEWMGGFIPIF GAANYAQKFQGRVTITADESTSTAYMELSSLRSDDTAVYYCARIPSGSYYYDYDMD VWGQGTTVTVSS |
| 4 | Heavy Chain Variable Region (VH) Nucleotide Sequence Anti-KIR mAb (IPH2102 / lirilumab) - (SEQ ID NO:18 from WO 2006/003179) |
| | caggtccagc tggtgcagtc tggggctgag gtgaagaagc ctgggtcctc ggtgaaggtc tcctgcaagg cttctggagg caccttcagt ttctatgcta tcagctgggt gcgacaggcc cctggacaag ggcttgagtg gatgggaggg ttcatcccta tctttggtgc agcaaactac gcacagaagt tccagggcag agtcacgatt accgcggacg aatccacgag cacagcctac atggaactga gcagcctgag atctgacgac acggccgtgt attactgtgc gagaatccct agtgggagct actactacga ctacgatatg gacgtctggg gccaagggac cacggtcacc gtctcctca |
| 5 | Light Chain Variable Region (VL) Amino Acid Sequence Anti-KIR mAb (IPH2102 / lirilumab) - (SEQ ID NO:15 from WO 2006/003179) |
| | EIVLTQSPVTLSLSPGERATLSCRASQSVSSYLAWYQQKPGQAPRLLIYD ASNRATGIPARFSGSGSGTDFTLTISSLEPEDFAVYYCQQRSNWMYTFGQ GTKLEIKRT |
| 6 | Light Chain Variable Region (VL) Nucleotide Sequence Anti-KIR mAb (IPH2102 / lirilumab) - (SEQ ID NO:16 from WO 2006/003179) |
| | gaaattgtgt tgacacagtc tccagtcacc ctgtctttgt ctccagggga aagagccacc ctctcctgca gggccagtca gagtgttagc agctacttag cctggtacca acagaaacct ggccaggctc ccaggctcct catctatgat gcatccaaca gggccactgg catcccagcc aggttcagtg gcagtgggtc tgggacagac ttcactctca ccatcagcag cctagagcct gaagattttg cagtttatta ttgtcagcag cgtagcaact ggatgtacac ttttggccag gggaccaagc tggagatcaa acgaact |
| 7 | Heavy Chain CDR1 Amino Acid Sequence Anti-KIR mAb (IPH2102 / lirilumab) - (from Figure 15 of WO 2006/003179) (corresponds to amino acid residues 31-35 of SEQ ID NO:1) |
| | FYAIS |
| 8 | Heavy Chain CDR2 Amino Acid Sequence Anti-KIR mAb (IPH2102 / lirilumab) - (from Figure 15 from WO 2006/003179) (corresponds to amino acid residues 50-65 of SEQ ID NO:1) |
| | GFIPIFGAANYAQKFQ |
| 9 | Heavy Chain CDR3 Amino Acid Sequence Anti-KIR mAb (IPH2102 / lirilumab) - (from Figure 15 from WO 2006/003179) (corresponds to amino acid residues 99-112 of SEQ ID NO:1) |
| | IPSGSYYYDYDMDV |
| 10 | Light Chain CDR1 Amino Acid Sequence Anti-KIR mAb (IPH2102 / lirilumab) - (from Figure 15 from WO 2006/003179) (corresponds to amino acid residues 24-34 of SEQ ID NO:3) |
| | RASQSVSSYLA |
| 11 | Light Chain CDR2 Amino Acid Sequence Anti-KIR mAb (IPH2102 / lirilumab) - (from Figure 15 from WO 2006/003179) (corresponds to amino acid residues 50-56 of SEQ ID NO:3) DASNRAT |
| 12 | Light Chain CDR3 Amino Acid Sequence Anti-KIR mAb (IPH2102 / lirilumab) - (from Figure 15 from WO 2006/003179) (corresponds to amino acid residues 89-97 of SEQ ID NO:3) |
| | QQRSNWMYT |
| 13 | KIR2DL1 Extracellular Domain (SEQ ID NO:23 from WO 2006/003179) |
| | HEGVHRKPSLLAHPGXLVKSEETVILQCWSDVMFEHFLLHREGMFNDT LRLIGEHHDGVSKANFSISRMTQDLAGTYRCYGSVTHSPYQVSAPSDPLD IVIIGLYEKPSLSAQXGPTVLAGENVTLSCSSRSSYDMYHLSREGEAHER RLPAGPKVNGTFQADFPLGPATHGGTYRCFGSFHDSPYEWSKSSDPLLVS VTGNPSNSWPSPTEPSSKTGNPRHLH |
| 14 | KIR2DL2 Extracellular Domain (SEQ ID NO:24 from WO 2006/003179) |
| | HEGVHRKPSLLAHPGRLVKSEETVILQCWSDVRFEHFLLHREGKFKDTLH LIGEHHDGVSKANFSIGPMMQDLAGTYRCYGSVTHSPYQLSAPSDPLDIV ITGLYEKPSLSAQPGPTVLAGESVTLSCSSRSSYDMYHLSREGEAHECRF SAGPKVNGTFQADFPLGPATHGGTYRCFGSFRDSPYEWSNSSDPLLVSVI GNPSNSWPSPTEPSSKTGNPRHLH |
| 15 | KIR2DL3 Extracellular Domain (SEQ ID NO:25 from WO 2006/003179) |
| | HEGVHRKPSLLAHPGPLVKSEETVILQCWSDVRFQHFLLHREGKFKDTLH LIGEHHDGVSKANFSIGPMMQDLAGTYRCYGSVTHSPYQLSAPSDPLDIV ITGLYEKPSLSAQPGPTVLAGESVTLSCSSRSSYDMYHLSREGEAHERRF SAGPKVNGTFQADFPLGPATHGGTYRCFGSFRDSPYEWSNSSDPLLVSVT GNPSNSWPSPTEPSSETGNPRHLH |
| 16 | KIR2DS4 Extracellular Domain (SEQ ID NO:38 from WO 2006/003179) |
| | QEGVHRKPSFLALPGHLVKSEETVILQCWSDVMFEHFLLHREGKFNNTLH LIGEHHDGVSKANFSIGPMMPVLAGTYRCYGSVPHSPYQLSAPSDPLDMV |
| 17 | Heavy Chain Amino Acid Sequence Anti-PD-1 mAb (BMS936558; 5C4 in WO 2006/121168) (variable region underlined; constant region bold) |
| | QVQLVESGGGVVQPGRSLRLDCKASGITFSNSGMHWVRQAPGKGL EWVAVIWYDGSKRYYADSVKGRFTISRDNSKNTLFLQMNSLRAEDT AVYYCATNDDYWGQGTLVTVSSASTKGPSVFPLAPCSRSTSESTAA LGCLVKDYFPEPVTVSWNSGALTSGVHTFPAVLQSSGLYSLSSVVT VPSSSLGTKTYTCNVDHKPSNTKVDKRVESKYGPPCPPCPAPEFLG |
| | GPSVFLFPPKPKDTLMISRTPEVTCVVVDVSQEDPEVQFNWYVDGV EVHNAKTKPREEQFNSTYRVVSVLTVLHQDWLNGKEYKCKVSNKG LPSSIEKTISKAKGQPREPQVYTLPPSQEEMTKNQVSLTCLVKGFYPS DIAVEWESNGQPENNYKTTPPVLDSDGSFFLYSRLTVDKSRWQEGNV FSCSVMHEALHNHYTQKSLSLSLGK |
| 18 | Light Chain Amino Acid Sequence Anti-PD-1 mAb (BMS936558; 5C4 in WO 2006/121168) (variable region underlined; constant region bold) |
| | EIVLTQSPATLSLSPGERATLSCRASQSVSSYLAWYQQKPGQAPRLLI YDASNRATGIPARFSGSGSGTDFTLTISSLEPEDFAVYYCQQSSNWPR TFGQGTKVEIKRTVAAPSVFIFPPSDEQLKSGTASVVCLLNNFYPREA KVQWKVDNALQSGNSQESVTEQDSKDSTYSLSSTLTLSKADYEKHK VYACEVTHQGLSSPVTKSFNRGEC |
| 19 | Heavy Chain Variable Region (VH) Amino Acid Sequence Anti-PD-1 mAb (BMS936558; 5C4 in WO 2006/121168) (SEQ ID NO:4 from WO 2006/121168) |
| | |
| 20 | Heavy Chain Variable Region (VH) Nucleotide Sequence Anti-PD-1 mAb (BMS936558; 5C4 in WO 2006/121168) (SEQ ID NO:60 from WO 2006/121168) |
| | cag gtg cag ctg gtg gag tct ggg gga ggc gtg gtc cag cct ggg agg tcc ctg aga ctc gac tgt aaa gcg tct gga atc acc ttc agt aac tct ggc atg cac tgg gtc cgc cag gct cca ggc aag ggg ctg gag tgg gtg gca gtt att tgg tat gat gga agt aaa aga tac tat gca gac tcc gtg aag ggc cga ttc acc atc tcc aga gac aat tcc aag aac acg ctg ttt ctg caa atg aac agc ctg aga gcc gag gac acg gct gtg tat tac tgt gcg aca aac gac gac tac tgg ggc cag gga acc ctg gtc acc gtc tcc tca |
| 21 | Light Chain Variable Region (VL) Amino Acid Sequence Anti-PD-1 mAb (BMS936558; 5C4 in WO 2006/121168) (SEQ ID NO:11 from WO 2006/121168) |
| | |
| 22 | Light Chain Variable Region (VL) Nucleotide Sequence Anti-PD-1 mAb (BMS936558; 5C4 in WO 2006/121168) (SEQ ID NO:67 from WO 2006/121168) |
| | gaa att gtg ttg aca cag tct cca gcc acc ctg tct ttg tct cca ggg gaa aga gcc acc ctc tcc tgc agg gcc agt cag agt |
| | gtt agt agt tac tta gcc tgg tac caa cag aaa cct ggc cag gct ccc agg ctc ctc atc tat gat gca tcc aac agg gcc act ggc atc cca gcc agg ttc agt ggc agt ggg tct ggg aca gac ttc act ctc acc atc agc agc cta gag cct gaa gat ttt gca gtt tat tac tgt cag cag agt agc aac tgg cct cgg acg ttc ggc caa ggg acc aag gtg gaa atc aaa |
| 23 | Heavy Chain CDR1 Amino Acid Sequence Anti-PD-1 mAb (BMS936558; 5C4 in WO 2006/121168) (SEQ ID NO:18 from WO 2006/121168) |
| | NSGMH |
| 24 | Heavy Chain CDR2 Amino Acid Sequence Anti-PD-1 mAb (BMS936558; 5C4 in WO 2006/121168) (SEQ ID NO:25 from WO 2006/121168) |
| | VIWYDGSKRYYADSVKG |
| 25 | Heavy Chain CDR3 Amino Acid Sequence Anti-PD-1 mAb (BMS936558; 5C4 in WO 2006/121168) (SEQ ID NO:32 from WO 2006/121168) |
| | NDDY |
| 26 | Light Chain CDR1 Amino Acid Sequence Anti-PD-1 mAb (BMS936558; 5C4 in WO 2006/121168) (SEQ ID NO:39 from WO 2006/121168) |
| | RASQSVSSYLA |
| 27 | Light Chain CDR2 Amino Acid Sequence Anti-PD-1 mAb (BMS936558; 5C4 in WO 2006/121168) (SEQ ID NO:46 from WO 2006/121168) |
| | DASNRAT |
| 28 | Light Chain CDR3 Amino Acid Sequence Anti-PD-1 mAb (BMS936558; 5C4 in WO 2006/121168) (SEQ ID NO:53 from WO 2006/121168) |
| | QQSSNWPRT |
| 29 | Complete PD-1 sequence (GenBank Accession No.: U64863) |
| | agtttccctt ccgctcacct ccgcctgagc agtggagaag gcggcactct ggtggggctg ctccaggcat gcagatccca caggcgccct ggccagtcgt ctgggcggtg ctacaactgg gctggcggcc aggatggttc ttagactccc cagacaggcc ctggaacccc cccaccttct tcccagccct gctcgtggtg |
| | accgaagggg acaacgccac cttcacctgc agcttctcca acacatcgga gagcttcgtg ctaaactggt accgcatgag ccccagcaac cagacggaca agctggccgc cttccccgag gaccgcagcc agcccggcca ggactgccgc ttccgtgtca cacaactgcc caacgggcgt gacttccaca tgagcgtggt cagggcccgg cgcaatgaca gcggcaccta cctctgtggg gccatctccc tggcccccaa ggcgcagatc aaagagagcc tgcgggcaga gctcagggtg acagagagaa gggcagaagt gcccacagcc caccccagcc cctcacccag gccagccggc cagttccaaa ccctggtggt tggtgtcgtg ggcggcctgc tgggcagcct ggtgctgcta gtctgggtcc tggccgtcat ctgctcccgg gccgcacgag ggacaatagg agccaggcgc accggccagc ccctgaagga ggacccctca gccgtgcctg tgttctctgt ggactatggg gagctggatt tccagtggcg agagaagacc ccggagcccc ccgtgccctg tgtccctgag cagacggagt atgccaccat tgtctttcct agcggaatgg gcacctcatc ccccgcccgc aggggctcag ccgacggccc tcggagtgcc cagccactga ggcctgagga tggacactgc tcttggcccc tctgaccggc ttccttggcc accagtgttc tgcagaccct ccaccatgag cccgggtcag cgcatttcct caggagaagc aggcagggtg caggccattg caggccgtcc aggggctgag ctgcctgggg gcgaccgggg ctccagcctg cacctgcacc aggcacagcc ccaccacagg actcatgtct caatgcccac agtgagccca ggcagcaggt gtcaccgtcc cctacaggga gggccagatg cagtcactgc ttcaggtcct gccagcacag agctgcctgc gtccagctcc ctgaatctct gctgctgctg ctgctgctgc tgctgctgcc tgcggcccgg ggctgaaggc gccgtggccc tgcctgacgc cccggagcct cctgcctgaa cttgggggct ggttggagat ggccttggag cagccaaggt gcccctggca gtggcatccc gaaacgccct ggacgcaggg cccaagactg ggcacaggag tgggaggtac atggggctgg ggactcccca ggagttatct gctccctgca ggcctagaga agtttcaggg aaggtcagaa gagctcctgg ctgtggtggg cagggcagga aacccctccc acctttacac atgcccaggc agcacctcag gccctttgtg gggcaggaa gctgaggcag taagcgggca ggcagagctg gaggcctttc aggccagcca gcactctggc ctcctgccgc cgcattccac cccagcccct cacaccactc gggagaggga catcctacgg tcccaaggtc aggagggcag ggctggggtt gactcaggcc cctcccagct gtggccacct gggtgttggg agggcagaag tgcaggcacc tagggccccc catgtgccca ccctgggagc tctccttgga acccattcct gaaattattt aaaggggttg gccgggctcc caccagggcc tgggtgggaa ggtacaggcg ttcccccggg gcctagtacc cccgcgtggc ctatccactc ctcacatcca cacactgcac ccccactcct ggggcagggc caccagcatc caggcggcca gcaggcacct gagtggctgg gacaaggat cccccttccc tgtggttcta ttatattata attataatta aatatgagag catgct |

## Claims

1. An anti-KIR antibody and an anti-PD-1 antibody for use in treating cancer,
wherein the anti-KIR antibody binds to KIR2DL1, KIR2DL2 and/or KIR2DL3 receptors and potentiates NK cell activity by reducing, neutralizing and/or reversing inhibition of NK cell cytotoxicity mediated by KIR2DL1, KIR2DL2 and/or KIR2DL3 receptors, and
wherein the anti-PD-1 antibody blocks the inhibitory activity of PD-1.

2. An anti-KIR antibody for use in treating cancer, wherein the treatment further comprises administration of an anti-PD-1 antibody,
wherein the anti-KIR antibody binds to KIR2DL1, KIR2DL2 and/or KIR2DL3 receptors and potentiates NK cell activity mediated by KIR2DL1, KIR2DL2 and/or KIR2DL3 receptors, and
wherein the anti-PD-1 antibody blocks the inhibitory activity of PD-1.

3. An anti-PD-1 antibody for use in treating cancer, wherein the treatment further comprises administration of an anti-KIR antibody,
wherein the anti-KIR antibody binds to KIR2DL1, KIR2DL2 and/or KIR2DL3 receptors and potentiates NK cell activity mediated by KIR2DL1, KIR2DL2 and/or KIR2DL3 receptors, and
wherein the anti-PD-1 antibody blocks the inhibitory activity of PD-1.

4. The antibody or antibodies for the use according to any one of claims 1-3, wherein said anti-KIR antibody does not bind to KIR2DS4 and/or KIR2DS3.

5. The antibody or antibodies for the use according to any one of claims 1-4, wherein the cancer is a solid tumor, in particular an advanced refractory solid tumor, or is a hematological malignancy.

6. The antibody or antibodies for the use according to any one of claims 1-5, wherein the cancer is chosen from:
(a) non-small cell lung cancer (NSCLC), such as squamous non-small cell lung cancer, renal cell carcinoma (RCC), melanoma, colorectal cancer, serous ovarian carcinoma, squamous cell cancer, cancer of the head or neck, skin cancer, cancer of the esophagus, cancer of the thyroid gland, cancer of the bladder, cancer of the urethra, cancer of the penis, carcinoma of the cervix, carcinoma of the vagina, carcinoma of the vulva, rectal cancer and cancer of the anal region; or
(b) a hematological malignancy, optionally chosen from multiple myeloma, Hodgkin lymphoma, follicular lymphoma, diffuse large B-cell lymphoma, and T-cell lymphoma.

7. The antibody or antibodies for the use according to any one of claims 1-5, wherein the cancer is cancer of the head or neck.

8. The antibody or antibodies for the use according to any one of claims 1-7, wherein:
(a) the anti-KIR antibody and the anti-PD-1 antibody are for being administered simultaneously in a single formulation; or
(b) the anti-KIR antibody and the anti-PD-1 antibody are formulated for separate administration; or
(c) the anti-KIR antibody and the anti-PD-1 antibody are for being administered concurrently; or
(d) the anti-KIR antibody and the anti-PD-1 antibody are for being administered sequentially; or
(e) the anti-PD-1 antibody is for being administered first followed by administration of the anti-KIR antibody; or
(f) the anti-KIR antibody is for being administered first followed by administration of the anti-PD-1 antibody.

9. The antibody or antibodies for the use according to any one of claims 1-8, wherein the anti-KIR antibody comprises a heavy chain variable region CDR1 having the sequence set forth in SEQ ID NO:7, a heavy chain variable region CDR2 having the sequence set forth in SEQ ID NO:8, a heavy chain variable region CDR3 having the sequence set forth in SEQ ID NO:9, a light chain variable region CDR1 having the sequence set forth in SEQ ID NO:10, a light chain variable region CDR2 having the sequence set forth in SEQ ID NO:11, and a light chain variable region CDR3 having the sequence set forth in SEQ ID NO:12.

10. The antibody or antibodies for the use according to claim 9, wherein the anti-KIR antibody comprises the heavy chain variable region having the sequence set forth in SEQ ID NO:3, and the light chain variable region having the sequence set forth in SEQ ID NO:5.

11. The antibody or antibodies for the use according to any one of claims 1-10, wherein the anti-PD-1 antibody comprises a heavy chain variable region CDR1 having the sequence set forth in SEQ ID NO:23, a heavy chain variable region CDR2 having the sequence set forth in SEQ ID NO:24, a heavy chain variable region CDR3 having the sequence set forth in SEQ ID NO:25, a light chain variable region CDR1 having the sequence set forth in SEQ ID NO:26, a light chain variable region CDR2 having the sequence set forth in SEQ ID NO:27, and a light chain variable region CDR3 having the sequence set forth in SEQ ID NO:28.

12. The antibody or antibodies for the use according to claim 11, wherein the anti-PD-1 antibody comprises the heavy chain variable region having the sequence set forth in SEQ ID NO:19, and the light chain variable region having the sequence set forth in SEQ ID NO:21.

13. A pharmaceutical composition for the use in treating cancer comprising:
(a) an anti-KIR antibody as recited in any one of claims 9 and 10;
(b) an anti-PD-1 antibody as recited in any one of claims 1-4, 11 and 12; and
(c) a pharmaceutically-acceptable carrier.

14. A kit for the use in treating cancer comprising:
(a) an anti-KIR antibody as recited in any one of claims 9 and 10;
(b) an anti-PD-1 antibody as recited in any one of claims 1-4,11 and 12; and
(c) a pharmaceutically acceptable carrier.

## Patentansprüche

1. Anti-KIR-Antikörper und Anti-PD-1-Antikörper zur Verwendung beim Behandeln von Krebs,
wobei der Anti-KIR-Antikörper an Rezeptoren KIR2DL1, KIR2DL2 und/oder KIR2DL3 bindet und NK-Zellaktivität potenziert durch Reduzieren, Neutralisieren und/oder Umkehren der Inhibition von NK-Zellzytotoxizität, die durch Rezeptoren KIR2DL1, KIR2DL2 und/oder KIR2DL3 vermittelt wird, und
wobei der Anti-PD-1-Antikörper die inhibitorische Aktivität von PD-1 blockiert.

2. Anti-KIR-Antikörper zur Verwendung beim Behandeln von Krebs, wobei die Behandlung ferner die Verabreichung eines Anti-PD-1-Antikörpers umfasst,
wobei der Anti-KIR-Antikörper an Rezeptoren KIR2DL1, KIR2DL2 und/oder KIR2DL3 bindet und durch Rezeptoren KIR2DL1, KIR2DL2 und/oder KIR2DL3 vermittelte NK-Zellaktivität potenziert, und
wobei der Anti-PD-1-Antikörper die inhibitorische Aktivität von PD-1 blockiert.

3. Anti-PD-1-Antikörper zur Verwendung beim Behandeln von Krebs, wobei die Behandlung ferner die Verabreichung eines Anti-KIR-Antikörpers umfasst,
wobei der Anti-KIR-Antikörper an Rezeptoren KIR2DL1, KIR2DL2 und/oder KIR2DL3 bindet und durch Rezeptoren KIR2DL1, KIR2DL2 und/oder KIR2DL3 vermittelte NK-Zellaktivität potenziert, und
wobei der Anti-PD-1-Antikörper die inhibitorische Aktivität von PD-1 blockiert.

4. Der oder die Antikörper zur Verwendung nach einem der Ansprüche 1-3, wobei der Anti-KIR-Antikörper nicht an KIR2DS4 und/oder KIR2DS3 bindet.

5. Der oder die Antikörper zur Verwendung nach einem der Ansprüche 1-4, wobei der Krebs ein fester Tumor, insbesondere ein fortgeschrittener refraktärer fester Tumor, oder eine hämatologische Malignität ist.

6. Der oder die Antikörper zur Verwendung nach einem der Ansprüche 1-5, wobei der Krebs ausgewählt ist aus:
(a) nicht kleinzelligem Lungenkrebs (NSCLC), wie squamösem nicht kleinzelligem Lungenkrebs, Nierenzellkarzinom (RCC), Melanom, Kolorektalkrebs, serösem Ovarialkarzinom, Plattenepithelkrebs, Krebs des Kopfes oder des Halses, Hautkrebs, Krebs der Speiseröhre, Krebs der Schilddrüse, Krebs der Blase, Krebs der Harnröhre, Krebs des Penis, Karzinom des Gebärmutterhalses, Karzinom der Vagina, Karzinom der Vulva, Rektalkrebs und Krebs der Analregion; oder
(b) einer hämatologischen Malignität, wahlweise ausgewählt aus multiplem Myelom, Hodgkin-Lymphom, follikulärem Lymphom, diffusem großzelligem B-Zell-Lymphom und T-Zell-Lymphom.

7. Der oder die Antikörper zur Verwendung nach einem der Ansprüche 1-5, wobei der Krebs Krebs des Kopfes oder des Halses ist.

8. Der oder die Antikörper zur Verwendung nach einem der Ansprüche 1-7, wobei:
(a) der Anti-KIR-Antikörper und der Anti-PD-1-Antikörper dazu bestimmt sind, simultan in einer einzigen Formulierung verabreicht zu werden; oder
(b) der Anti-KIR-Antikörper und der Anti-PD-1-Antikörper zwecks separater Verabreichung formuliert sind; oder
(c) der Anti-KIR-Antikörper und der Anti-PD-1-Antikörper dazu bestimmt sind, gleichzeitig verabreicht zu werden; oder
(d) der Anti-KIR-Antikörper und der Anti-PD-1-Antikörper dazu bestimmt sind, sequenziell verabreicht zu werden; oder
(e) der Anti-PD-1-Antikörper dazu bestimmt ist, zuerst verabreicht zu werden, gefolgt von der Verabreichung des Anti-KIR-Antikörpers; oder
(f) der Anti-KIR-Antikörper dazu bestimmt ist, zuerst verabreicht zu werden, gefolgt von der Verabreichung des Anti-PD-1-Antikörpers.

9. Der oder die Antikörper zur Verwendung nach einem der Ansprüche 1-8, wobei der Anti-KIR-Antikörper eine variable Schwerkettenregion CDR1 mit der in SEQ ID NO: 7 aufgeführten Sequenz, eine variable Schwerkettenregion CDR2 mit der in SEQ ID NO: 8 aufgeführten Sequenz, eine variable Schwerkettenregion CDR3 mit der in SEQ ID NO: 9 aufgeführten Sequenz, eine variable Leichtkettenregion CDR1 mit der in SEQ ID NO: 10 aufgeführten Sequenz, eine variable Leichtkettenregion CDR2 mit der in SEQ ID NO: 11 aufgeführten Sequenz und eine variable Leichtkettenregion CDR3 mit der in SEQ ID NO: 12 aufgeführten Sequenz umfasst.

10. Der oder die Antikörper zur Verwendung nach Anspruch 9, wobei der Anti-KIR-Antikörper die variable Schwerkettenregion mit der in SEQ ID NO: 3 aufgeführten Sequenz und die variable Leichtkettenregion mit der in SEQ ID NO: 5 aufgeführten Sequenz umfasst.

11. Der oder die Antikörper zur Verwendung nach einem der Ansprüche 1-10, wobei der Anti-PD-1-Antikörper eine variable Schwerkettenregion CDR1 mit der in SEQ ID NO: 23 aufgeführten Sequenz, eine variable Schwerkettenregion CDR2 mit der in SEQ ID NO: 24 aufgeführten Sequenz, eine variable Schwerkettenregion CDR3 mit der in SEQ ID NO: 25 aufgeführten Sequenz, eine variable Leichtkettenregion CDR1 mit der in SEQ ID NO: 26 aufgeführten Sequenz, eine variable Leichtkettenregion CDR2 mit der in SEQ ID NO: 27 aufgeführten Sequenz und eine variable Leichtkettenregion CDR3 mit der in SEQ ID NO: 28 aufgeführten Sequenz umfasst.

12. Der oder die Antikörper zur Verwendung nach Anspruch 11, wobei der Anti-PD-1-Antikörper die variable Schwerkettenregion mit der in SEQ ID NO: 19 aufgeführten Sequenz und die variable Leichtkettenregion mit der in SEQ ID NO: 21 aufgeführten Sequenz umfasst.

13. Pharmazeutische Zusammensetzung zur Verwendung beim Behandeln von Krebs, umfassend:
(a) einen Anti-KIR-Antikörper gemäß einem der Ansprüche 9 und 10;
(b) einen Anti-PD-1-Antikörper gemäß einem der Ansprüche 1-4, 11 und 12; und
(c) einen pharmazeutisch verträglichen Träger.

14. Kit zur Verwendung beim Behandeln von Krebs, umfassend:
(a) einen Anti-KIR-Antikörper gemäß einem der Ansprüche 9 und 10;
(b) einen Anti-PD-1-Antikörper gemäß einem der Ansprüche 1-4, 11 und 12; und
(c) einen pharmazeutisch verträglichen Träger.

## Revendications

1. Anticorps anti-KIR et anticorps anti-PD-1 pour utilisation dans le traitement d'un cancer,
dans lequel l'anticorps anti-KIR se lie aux récepteurs KIR2DL1, KIR2DL2 et/ou KIR2DL3 et potentialise l'activité de cellules NK par la réduction, la neutralisation et/ou l'inversion de l'inhibition d'une cytotoxicité de cellule NK induite par des récepteurs KIR2DL1, KIR2DL2 et/ou KIR2DL3, et
dans lequel l'anticorps anti-PD-1 bloque l'activité inhibitrice de PD-1.

2. Anticorps anti-KIR pour utilisation dans le traitement d'un cancer, dans lequel le traitement comprend en outre l'administration d'un anticorps anti-PD-1,
dans lequel l'anticorps anti-KIR se lie aux récepteurs KIR2DL1, KIR2DL2 et/ou KIR2DL3 et potentialise l'activité de cellules NK induite par des récepteurs KIR2DL1, KIR2DL2 et/ou KIR2DL3, et
dans lequel l'anticorps anti-PD-1 bloque l'activité inhibitrice de PD-1.

3. Anticorps anti-PD-1 pour utilisation dans le traitement d'un cancer, dans lequel le traitement comprend en outre l'administration d'un anticorps anti-KIR,
dans lequel l'anticorps anti-KIR se lie aux récepteurs KIR2DL1, KIR2DL2 et/ou KIR2DL3 et potentialise l'activité de cellules NK induite par les récepteurs KIR2DL1, KIR2DL2 et/ou KIR2DL3, et
dans lequel l'anticorps anti-PD-1 bloque l'activité inhibitrice de PD-1.

4. L'anticorps ou les anticorps pour l'utilisation selon l'une quelconque des revendications 1-3, dans lequel ledit anticorps anti-KIR ne se lie pas à KIR2DS4 et/ou KIR2DS3.

5. L'anticorps ou les anticorps pour l'utilisation selon l'une quelconque des revendications 1-4, dans lequel le cancer est une tumeur solide, en particulier une tumeur solide réfractaire avancée, ou est une malignité hématologique.

6. L'anticorps ou les anticorps pour l'utilisation selon l'une quelconque des revendications 1-5, dans lequel le cancer est choisi parmi:
(a) un cancer du poumon non à petites cellules (NSCLC), tel qu'un cancer du poumon non à petites cellules squameux, un carcinome des cellules rénales (RCC), un mélanome, un cancer colorectal, un carcinome ovarien séreux, un cancer des cellules squameuses, un cancer de la tête ou du cou, un cancer de la peau, un cancer de l'œsophage, un cancer de la glande thyroïde, un cancer de la vessie, un cancer de l'urètre, un cancer du pénis, un carcinome du col de l'utérus, un carcinome du vagin, un carcinome de la vulve, un cancer rectal et un cancer de la région anale; ou
(b) une malignité hématologique, optionnellement choisie parmi un myélome multiple, un lymphome hodgkinien, un lymphome folliculaire, un lymphome diffus à grande cellules Bet un lymphome à cellules T.

7. L'anticorps ou les anticorps pour l'utilisation selon l'une quelconque des revendications 1-5, dans lequel le cancer est un cancer de la tête ou du cou.

8. L'anticorps ou les anticorps pour l'utilisation selon l'une quelconque des revendications 1-7, dans lequel:
(a) l'anticorps anti-KIR et l'anticorps anti-PD-1 sont destinés à être administrés simultanément dans une seule formulation; ou
(b) l'anticorps anti-KIR et l'anticorps anti-PD-1 sont formulés pour une administration séparée; ou
(c) l'anticorps anti-KIR et l'anticorps anti-PD-1 sont destinés à être administrés concurremment; ou
(d) l'anticorps anti-KIR et l'anticorps anti-PD-1 sont destinés à être administrés séquentiellement; ou
(e) l'anticorps anti-PD-1 est destiné à être administré en premier suivi par l'administration de l'anticorps anti-KIR; ou
(f) l'anticorps anti-KIR est destiné à être administré en premier suivi par l'administration de l'anticorps anti-PD-1.

9. L'anticorps ou les anticorps pour l'utilisation selon l'une quelconque des revendications 1-8, dans lequel l'anticorps anti-KIR comprend une CDR1 de région variable de chaîne lourde possédant la séquence présentée dans la SEQ ID NO:7, une CDR2 de région variable de chaîne lourde possédant la séquence présentée dans la SEQ ID NO:8, une CDR3 de région variable de chaîne lourde possédant la séquence présentée dans la SEQ ID NO:9, une CDR1 de région variable de chaîne légère possédant la séquence présentée dans la SEQ ID NO:10, une CDR2 de région variable de chaîne légère possédant la séquence présentée dans la SEQ ID NO:11 et une CDR3 de région variable de chaîne légère possédant la séquence présentée dans la SEQ ID NO:12.

10. L'anticorps ou les anticorps pour l'utilisation selon la revendication 9, dans lequel l'anticorps anti-KIR comprend la région variable de chaîne lourde possédant la séquence présentée dans la SEQ ID NO:3 et la région variable de chaîne légère possédant la séquence présentée dans la SEQ ID NO:5.

11. L'anticorps ou les anticorps pour l'utilisation selon l'une quelconque des revendications 1-10, dans lequel l'anticorps anti-PD-1 comprend une CDR1 de région variable de chaîne lourde possédant la séquence présentée dans la SEQ ID NO:23, une CDR2 de région variable de chaîne lourde possédant la séquence présentée dans la SEQ ID NO:24, une CDR3 de région variable de chaîne lourde possédant la séquence présentée dans la SEQ ID NO:25, une CDR1 de région variable de chaîne légère possédant la séquence présentée dans la SEQ ID NO:26, une CDR2 de région variable de chaîne légère possédant la séquence présentée dans la SEQ ID NO:27 et une CDR3 de région variable de chaîne légère possédant la séquence présentée dans la SEQ ID NO:28.

12. L'anticorps ou les anticorps pour l'utilisation selon la revendication 11, dans lequel l'anticorps anti-PD-1 comprend la région variable de chaîne lourde possédant la séquence présentée dans la SEQ ID NO:19 et la région variable de chaîne légère possédant la séquence présentée dans la SEQ ID NO:21.

13. Composition pharmaceutique pour l'utilisation dans le traitement du cancer comprenant:
(a) un anticorps anti-KIR selon l'une quelconque des revendications 9 et 10;
(b) un anticorps anti-PD-1 selon l'une quelconque des revendications 1-4, 11 et 12; et
(c) un véhicule pharmaceutiquement acceptable.

14. Kit pour l'utilisation dans le traitement du cancer comprenant:
(a) un anticorps anti-KIR selon l'une quelconque des revendications 9 et 10;
(b) un anticorps anti-PD-1 selon l'une quelconque des revendications 1-4, 11 et 12; et
(c) un véhicule pharmaceutiquement acceptable.
